# EUROPEAN PATENT APPLICATION

(11) **EP 4 821 951 A1**
(43) Date of publication of application: **14.10.2026**
(21) Application number: 24899962.5
(22) Date of filing: 06.12.2024
(51) Int. Cl.: C07K 16/46, A61P 35/00, A61K 39/395, C07K 16/30, A61K 47/68

(54) **ANTI-C5 AND HTRA1 BISPECIFIC ANTIBODY AND USE THEREOF**

(30) Priority: 07.12.2023 CN 202311675539; 29.02.2024 CN 202410228514
(71) Applicant: Innovent Biologics (Suzhou) Co., Ltd., Suzhou, Jiangsu 215123 (CN)
(72) Inventor: LI, Yiming, Suzhou, Jiangsu 215123 (CN); XIA, Huan, Suzhou, Jiangsu 215123 (CN); BIAN, Yan, Suzhou, Jiangsu 215123 (CN); LI, Li, Suzhou, Jiangsu 215123 (CN)
(74) Representative: Sagittarius IP
(86) International application number: PCT/CN2024/137414
(87) International publication number: WO 2025/119329

(57) **Abstract**

The present invention relates to a novel anti-C5 antibody and a novel HTRA1 antibody, and a bispecific antibody specifically binding C5 and HTRA1 constructed based thereof. In particular, the invention relates to an anti-C5 antibody specifically binding a different epitope of C5 or a bispecific antibody specifically binding a different epitope of C5 and HTRA1. The invention also relates to a nucleic acid encoding said antibody, an expression vector comprising said nucleic acid, and a host cell comprising said nucleic acid or expression vector. The invention also relates to pharmaceutical compositions or pharmaceutical combinations comprising the antibodies, and the invention further relates to methods of treating diseases and uses based on the antibodies.

## Description

### CROSS-REFERENCE TO RELATED APPLICATIONS

This application is based on and claims priority to the Chinese Patent Application with the application number of 202311675539.2, the filing date of Dec. 07, 2023, and the Chinese Patent Application with the application number of 20241228514.6, the filing date of Feb. 29, 2024, the entire contents of which are hereby incorporated herein by reference.

The present invention relates to a novel anti-C5 antibody and a novel HTRA1 antibody, and a bispecific antibody specifically binding C5 and HTRA1 constructed based thereof. In particular, the invention relates to an anti-C5 antibody specifically binding a different epitope of C5 or a bispecific antibody specifically binding a different epitope of C5 and HTRA1. The invention also relates to a nucleic acid encoding said antibody, an expression vector comprising said nucleic acid, and a host cell comprising said nucleic acid or expression vector. The invention also relates to pharmaceutical compositions or pharmaceutical combinations comprising the antibodies, and the invention further relates to methods of treating diseases and uses based on the antibodies.

### Background of the Invention

The complement system is an essential component of the innate immunity in humans and, when activated, it causes target cell lysis and promotes phagocytosis through opsonization. Complement is activated through a series of proteolytic steps through three major pathways: the classical pathway, which is typically activated by immune complexes; the alternative pathway, which can be induced by unprotected cell surfaces; and the mannose-binding lectin pathway. All three pathways of the complement cascade are focused on proteolytic cleavage of complement component 5 (C5) proteins. Cleavage of complement component 5 (C5) results in the production of fragments C5a and C5b, which are critical in the activation of the complement cascade. C5a can produce pleiotropic physiologic responses by binding to its receptor. C5a is a potent pro-inflammatory mediator that induces chemotactic migration, enhances cell adhesion, stimulates oxidative burst, and induces the release of various inflammatory mediators such as histamine or cytokines. C5b mediates the formation of the membrane attack complex (MAC, or C5b-9), which leads to cell lysis in the late stages of complement-dependent cytotoxicity (CDC). Furthermore, in nucleated cells resistant to C5b-9 cytolysis, sub-lytic amounts of C5b-9 cause cellular activation leading to cell proliferation, production of proinflammatory mediators, and extracellular matrix production.

The serine protease HtrA1 serine peptidase 1 (HtrA1) (PRSS11; clan PA, family 51) belongs to an evolutionarily conserved family of HtrA proteins. In humans, HtrA1, HtrA3, and HtrA4 share the same domain structure: N-terminal IGFBP-like and Kazal-like components, a protease domain with a trypsin-like fold, and a C-terminal PDZ domain. The physiological relevance of HtrA1 has been reliably confirmed by the identification of human loss-of-function mutations that cause familial ischemic cerebral small-vessel disease. The molecular mechanism involves defective TGFβ inhibition by HtrA1 leading to increased TGFβ signaling. Dysregulated TGFβ signaling via aberrant HtrA1 expression may also lead to arthritic disease in conjunction with HtrA1-mediated degradation of various extracellular matrix components or indirectly via upregulation of matrix metalloproteinases. Additionally, human genetic studies confirmed a strong correlation between the development of age-related macular degeneration (AMD) and SNPs in the HtrA1 promoter region that result in increased levels of HtrA1 transcription.

Antibodies to C5 have been developed in the prior art, e.g., eculizumab. However, in the in vitro hemolysis inhibition activity test, although eculizumab showed a good hemolytic inhibition effect of the classical pathway, its insufficient activity to block the alternative complement pathway (AP) did not fully meet the needs of patients. For example, some PNH patients treated with eculizumab (Soliris) were unable to get rid of transfusion dependence due to extravascular hemolysis.

Combinations of different C5 antibodies (WO2019118556A1) and antibodies specifically binding HTRA1 (WO2017075212A1) have also been developed in the prior art.

However, the blockade of the complement pathway by existing anti-C5 antibodies is still insufficient, and the therapeutic effect needs to be improved. At the same time, multiple injections are required for the combination therapy, which brings a burden to patients.

Accordingly, there exists development of therapeutic agents that are capable of greater blocking activity, better therapeutic efficacy, and simplified administration.

### Summary Of The Invention

In one aspect, novel VHH antibodies specifically binding C5 and heavy chain antibodies comprising thereof are developed. The invention also develops novel VHH antibodies specifically binding HTRA1 and heavy chain antibodies comprising thereof.

In another aspect, the invention developed an anti-C5 antibody based on the VHH of anti-C5 specifically binding a different epitope of C5.

In another aspect, the invention developed a multispecific binding protein based on the VHH of anti-C5 and the VHH of anti-HTRA1, e.g. a bispecific antibody specifically binding C5 as well as HTRA1, particularly, specifically binds to different epitopes of C5, and also specifically binds HTRA1.

In an embodiment, a bispecific antibody of the invention has one or more of the following properties:
(1) It blocks both human C5 and human HTRA1;
(2) It has a stronger complement pathway blocking pathway, for example, it can completely block the complement alternative pathway;
(3) It showes better therapeutic effect than single-target drugs in the sodium iodate-induced geographic atrophy model;
(4) A single dose can achieve both anti-C5 and anti-HTRA1 effects.

In some embodiments, the present invention relates to the following specific embodiments:
1. A VHH antibody specifically binding C5, comprising
   three complementarity determining regions (CDRs) contained in the VHH of any one of SEQ ID NOs: 23, 17 or 22;
   preferably, the CDR1 sequence is defined according to AbM, CDR2 is defined according to Kabat, and CDR3 is defined according to AbM.
2. The VHH antibody specifically binding C5 of embodiment 1, comprising complementarity determining regions (CDRs) VHH CDR1, VHH CDR2 and VHH CDR3, wherein
   (i) VHH CDR1 comprises or consists of the amino acid sequence set forth in SEQ ID NO:1, VHH CDR2 comprises or consists of the amino acid sequence set forth in SEQ ID NO:9, VHH CDR3 comprises or consists of the amino acid sequence set forth in SEQ ID NO:10;
   (ii) VHH CDR1 comprises or consists of the amino acid sequence set forth in SEQ ID NO:1, VHH CDR2 comprises or consists of the amino acid sequence set forth in SEQ ID NO:2, VHH CDR3 comprises or consists of the amino acid sequence set forth in SEQ ID NO:3; or
   (iii) VHH CDR1 comprises or consists of the amino acid sequence set forth in SEQ ID NO:1, VHH CDR2 comprises or consists of the amino acid sequence set forth in SEQ ID NO:2 or 9, VHH CDR3 comprises or consists of the amino acid sequence set forth in SEQ ID NO:3 or 10.
3. The VHH antibody specifically binding C5 of embodiment 1, comprising or consisting of a heavy chain variable region, which
   (i) comprises or consists of an amino acid sequence having at least 90%, 91%, 92%, 93%, 94%, 95%, 96%, 97%, 98% or 99% identity to an amino acid sequence selected from any one set forth in SEQ ID NOs: 23, 17 or 22; or
   (ii) comprises or consists of an amino acid sequence selected from any one set forth in SEQ ID NOs: 23, 17 or 22.
4. A VHH antibody specifically binding C5, comprising
   three complementarity determining regions (CDRs) contained in the VHH of any one of SEQ ID NOs: 20, 18, 19 or 21,
   preferably, the CDR1 sequence is defined according to AbM, CDR2 is defined according to Kabat, and CDR3 is defined according to AbM.
5. The VHH antibody specifically binding C5 of embodiment 4, comprising complementarity determining regions (CDRs) VHH CDR1, VHH CDR2 and VHH CDR3, wherein
   (i) VHH CDR1 comprises or consists of the amino acid sequence set forth in SEQ ID NO:4, VHH CDR2 comprises or consists of the amino acid sequence set forth in SEQ ID NOs:7 or 8, VHH CDR3 comprises or consists of the amino acid sequence set forth in SEQ ID NO:6;
   (ii) VHH CDR1 comprises or consists of the amino acid sequence set forth in SEQ ID NO:4, VHH CDR2 comprises or consists of the amino acid sequence set forth in SEQ ID NO:5, VHH CDR3 comprises or consists of the amino acid sequence set forth in SEQ ID NO:6 or 30; or
   (iii) VHH CDR1 comprises or consists of the amino acid sequence set forth in SEQ ID NO:4, VHH CDR2 comprises or consists of the amino acid sequence set forth in SEQ ID NOs:5, 7 or 8, VHH CDR3 comprises or consists of the amino acid sequence set forth in SEQ ID NOs:30 or 6.
6. The VHH antibody specifically binding C5 of embodiment 4, comprising or consisting of a heavy chain variable region, which
   (i) comprises or consists of an amino acid sequence that is at least 90%, 91%, 92%, 93%, 94%, 95%, 96%, 97%, 98% or 99% identical to an amino acid sequence selected from any one set forth in SEQ ID NOs: 20, 18, 19 or 21; or
   (ii) comprises or consists of an amino acid sequence selected from any one set forth in SEQ ID NOs: 20, 18, 19 or 21.
7. A heavy chain antibody specifically binding C5, comprising the VHH antibody according to any one of embodiments 1-6.
8. The heavy chain antibody specifically binding C5 of embodiment 7, comprising or consisting of the VHH antibody specifically binding C5 of any one of embodiments 1-6 linked to an antibody constant region or Fc region.
9. A VHH antibody specifically binding HTRA1 comprising
   three complementarity determining regions (CDRs) contained in the VHH set forth in SEQ ID NO: 25 or 24
   preferably, the CDR1 sequence is defined according to AbM, CDR2 is defined according to Kabat, and CDR3 is defined according to AbM.
10. The VHH antibody specifically binding HTRA1 of embodiment 9, comprising complementarity determining regions (CDRs) VHH CDR1, VHH CDR2 and VHH CDR3, wherein
   (i) VHH CDR1 comprises or consists of the amino acid sequence set forth in SEQ ID NO:14, VHH CDR2 comprises or consists of the amino acid sequence set forth in SEQ ID NO:15, VHH CDR3 comprises or consists of the amino acid sequence set forth in SEQ ID NO:16; or
   (ii) VHH CDR1 comprises or consists of the amino acid sequence set forth in SEQ ID NO:11, VHH CDR2 comprises or consists of the amino acid sequence set forth in SEQ ID NO:12, VHH CDR3 comprises or consists of the amino acid sequence set forth in SEQ ID NO:13; or
   (iii) VHH CDR1 comprises or consists of the amino acid sequence set forth in SEQ ID NO:11 or 14, VHH CDR2 comprises or consists of the amino acid sequence set forth in SEQ ID NO:12 or 15, VHH CDR3 comprises or consists of the amino acid sequence set forth in SEQ ID NO:13 or 16.
11. The VHH antibody specifically binding HTRA1 of embodiment 9, comprising or consisting of a heavy chain variable region, which
   (i) comprises or consists of an amino acid sequence having at least 90%, 91%, 92%, 93%, 94%, 95%, 96%, 97%, 98% or 99% identity to amino acid sequence selected from any one set forth in SEQ ID NOs: 25 or 24; or
   (ii) comprises or consists of amino acid sequence selected from any one set forth in SEQ ID NOs: 25 or 24.
12. A heavy chain antibody specifically binding HTRA1 comprising the VHH antibody specifically binding HTRA1 according to any one of embodiments 9-11.
13. The heavy chain antibody specifically binding HTRA1 of embodiment 12, comprising or consisting of the VHH antibody specifically binding HTRA1 of any one of embodiments 9-11 linked to an antibody constant region or Fc region.
14. The heavy chain antibody specifically binding C5 of embodiment 8 or the heavy chain antibody specifically binding HTRA1 of embodiment 13, wherein the antibody constant region or Fc region is from human IgG1, human IgG2, human IgG3 or human IgG4.
15. The VHH antibody specifically binding C5 of any one of embodiments 1-6, or the heavy chain antibody specifically binding C5 of embodiments 7 or 8, or the VHH antibody specifically binding HTRA1 of any one of embodiments 9-11, or the heavy chain antibody specifically binding HTRA1 of embodiments 12 or 13, or the heavy chain antibody specifically binding C5 or the heavy chain antibody specifically binding HTRA1 of embodiment 14, wherein the antibody is a chimeric antibody or a humanized antibody.
16. An antibody specifically binding C5 comprising two or three or more antigen-binding regions that specifically bind C5, wherein the antigen-binding regions bind distinct epitopes and comprise the VHH antibody specifically binding C5 of any one of claims 1-6 and 15, or the heavy chain antibody specifically binding C5 of any one of embodiments 7-8 and 14-15, respectively.
17. The antibody specifically binding C5 of embodiment 16, comprising 1 or 2 VHH antibodies or heavy chain antibodies comprising the VHH antibody specifically binding C5 according to any one of embodiments 1-3, and 1 or 2 VHH antibodies or heavy chain antibodies comprising the VHH antibody specifically binding C5 according to any one of embodiments 4-6, preferably, comprising 1 or 2 VHH antibodies that specifically binding C5 according to any one of embodiments 4-6, and 1 VHH antibody specifically binding C5 according to any one of embodiments 1-3, preferably, the VHH antibodies or the heavy chain antibodies are connected in tandem, preferably via a linker;
   preferably, the antibody comprises or consists of a chain comprising from the N-terminus to the C-terminus:
      (1) the VHH antibody specifically binding C5 of any one of embodiments 4-6 - the VHH antibody specifically binding C5 of any one of embodiments 1-3,
      (2) the VHH antibody specifically binding C5 of any one of embodiments 4-6 - the VHH antibody specifically binding C5 of any one of embodiments 4-6 - the VHH antibody specifically binding C5 of any one of embodiments 1-3, or
      (3) the VHH antibody specifically binding C5 of any one of embodiments 4-6 - the VHH antibody specifically binding C5 of any one of embodiments 1-3 - the VHH antibody specifically binding C5 of any one of embodiments 4-6;
   preferably, the VHHs are linked by a linker.
18. A multispecific antibody comprising one or more antigen-binding regions which specifically bind C5 and one or more other antigen-binding regions, where the antigen-binding region specifically binding C5 comprises the VHH antibody specifically binding C5 of any one of embodiments 1-6 and 15 or the heavy chain antibody specifically binding C5 of any one of embodiments 7-8 and 14-15, preferably, the multispecific antibody is a bispecific antibody.
19. The multispecific antibody of embodiment 18 further comprising one or more antigen-binding regions which specifically bind HTRA1, for example the antigen-binding region which specifically bind HTRA1 comprises the VHH antibody specifically binding HTRA1 of any one of embodiments 9-11 and 15, or the heavy chain antibody specifically binding HTRA1 of any one of embodiments 12-15.
20. A multispecific antibody comprising an antigen-binding region specifically binding HTRA1 and one or more other antigen-binding regions, wherein the antigen-binding region specifically binding HTRA1 comprises the VHH antibody specifically binding HTRA1 of any one of embodiments 9-11 and 15, or the heavy chain antibody specifically binding HTRA1 of any one of embodiments 12-15, preferably, the multispecific antibody is a bispecific antibody.
21. The multispecific antibody of embodiment 20 further comprising one or more antigen-binding regions which specifically bind C5, wherein the antigen-binding regions which specifically bind C5 comprise the VHH antibody specifically binding C5 of any one of embodiments 1-6 and 15, or the heavy chain antibody specifically binding C5 of any one of embodiments 7-8 and 14-15.
22. A bispecific antibody comprising one or more antigen-binding regions which specifically bind C5 and one or more antigen-binding regions which specifically bind HTRA1, wherein the antigen-binding regions which specifically bind C5 bind different epitopes on C5.
23. The bispecific antibody of embodiment 22 comprising two antigen-binding regions which specifically bind C5 and one antigen-binding region specifically binding HTRA1, wherein the two antigen-binding regions which specifically bind C5 bind different epitopes on C5.
24. The bispecific antibody of embodiment 23 wherein the two antigen-binding regions specifically binding C5 are VHH specifically binding different epitopes of C5 and the antigen-binding region specifically binding HTRA1 is VHH specifically binding HTRA1, wherein the 3 VHHs are connected in tandem, optionally the VHHs are linked together by a linker.
25. The bispecific antibody of embodiment 24 wherein the linker comprises (G) ₙ, where n = 5-10, for example 5, 6, 7, 8, 9, or 10.
26. The bispecific antibody of embodiment 22 or 23 wherein the VHHs are linked to each other via an N-terminus to an N-terminus, a C-terminus to a C-terminus, or an N-terminus to a C-terminus, optionally via a linker.
27. The bispecific antibody of any one of embodiments 24-26 wherein the one antigen-binding region specifically binding C5 comprises or consists of the VHH of any one of embodiments 1-3, the other antigen-binding region specifically binding C5 comprises or consists of the VHH of any one of embodiments 4-6 or the antigen-binding region specifically binding HTRA1 comprises or consists of the VHH of any one of embodiments 9-11.
28. The bispecific antibody of any one of embodiments 24-26 wherein the one antigen-binding region specifically binding C5 comprises or consists of the VHH of any one of embodiments 1-3, the other antigen-binding region specifically binding C5 comprises or consists of the VHH of any one of embodiments 4-6, and the antigen-binding region specifically binding HTRA1 comprises or consists of the VHH of any one of embodiments 9-11.
29. The bispecific antibody of any one of embodiments 24-28 comprising a chain which comprises, from N-terminus to C-terminus:
   VHH1 specifically binding C5-VHH2 specifically binding C5-VHH3 specifically binding HTRA1,
   the VHHs are linked by a linker;
   wherein VHH1 comprises or consists of the VHH of any one of embodiments 4-6, VHH2 comprises or consists of the VHH of any one of embodiments 1-3, and VHH3 comprises or consists of the VHH of any one of embodiments 9-11.
30. The bispecific antibody of embodiment 29 comprising or consisting of a chain wherein the chain comprises or consists of the amino acid sequence set forth in SEQ ID NO:27 or an amino acid sequence having at least 90%, 91%, 92%, 93%, 94%, 95%, 96%, 97%, 98% or 99% identity thereto.
31. A nucleic acid molecule encoding the VHH antibody specifically binding C5 of any one of embodiments 1-6 and 15, or the heavy chain antibody specifically binding C5 of any one of embodiments 7 or 8 or 15, or the VHH antibody specifically binding HTRA1 of any one of embodiments 9-11, or the heavy chain antibody specifically binding HTRA1 of any one of embodiments 12 or 13 or 15, or the heavy chain antibody specifically binding C5 or the heavy chain antibody specifically binding HTRA1 of any one of embodiments 14 or 15; or the multispecific antibody of any one of embodiments 17-20, or the bispecific antibody of any one of embodiments 21-28.
32. An expression vector comprising the nucleic acid molecule according to embodiment 31, preferably, the expression vector is pCDNA3.1.
33. A host cell comprising the nucleic acid molecule according to embodiment 31 or the expression vector according to embodiment 32, preferably, the host cell is a prokaryotic or eukaryotic, for example, a CHO cell, a 293 cell, for example, an Expi293 cell.
34. A method of producing the antibody of any one of embodiments 1-30, comprising culturing the host cell of embodiment 33 under conditions suitable for the expression of the antibody or fusion protein, and optionally further comprising isolating the protein from the host cell or the host cell culture medium, and/or purifying the protein.
35. An immunoconjugate comprising the antibody of any one of embodiments 1-30, and one or more other active ingredients.
36. A pharmaceutical composition or a medicament or a formulation comprising the antibody of any one of embodiments 1-30 or the immunoconjugate of embodiment 35, and optionally a pharmaceutical excipient.
37. A pharmaceutical combination product, comprising
   the antibody of any one of embodiments 1-30 or the immunoconjugate of embodiment 35; and
   other therapeutic agents.
38. A method of preventing or treating an ocular disease or condition in a subject, comprising administering to the subject an effective amount of the antibody of any one of embodiments 1-30; or the immunoconjugate of embodiment 35 or the pharmaceutical composition or formulation of embodiment 36; or the pharmaceutical combination product of embodiment 37.
39. The method of embodiment 38, wherein the disease or condition refers to a disease or condition in which complement C5 protein and/or HTRA1 protein is present (e.g., at an elevated level, e.g., of nucleic acid or protein) in the subject (e.g., as compared to a healthy subject) or in a sample, e.g., a body fluid, of the subject (e.g., as compared to a body fluid of a healthy subject).
40. A method for detecting the presence of complement C5 and/or HTRA1 in a biological sample comprising contacting a biological sample with the antibody of any one of embodiments 1-30 under conditions that allow binding thereof to complement C5 and/or HTRA1 and detecting whether a complex is formed between the antibody and complement C5 and/or HTRA1, wherein the formation of the complex is indicative of the presence of complement C5.

### Brief Description of the Drawing

Figure 1 shows the results of an anti-C5 VHH antibody blocking complement classical pathway induced hemolysis experiment.
Figure 2 shows the results of an anti-C5 VHH antibody blocking the complement alternative pathway induced hemolysis experiment.
Figure 3 shows the results of anti-C5 VHH antibodies blocking the production of C5a in the complement classical pathway (C5a ELISA).
Figure 4 shows the results of the bin assay for the anti-C5 VHH antibodies.
Figure 5 shows the results of the anti-HTRA1 VHH antibodies blocking H2-Opt assay.
Figure 6 shows the results of the anti-HTRA1 VHH antibodies blocking elastase assay.
Figure 7 shows the results of hemolysis experiment induced by humanized anti-C5 VHH antibody blocking the complement classical pathway and hemolysis experiment induced by blocking the complement alternative pathway.
Figure 8 shows the results of an affinity matured anti-HTRA1 VHH antibody blocking H2-Opt assay.
Figure 9 shows the results of the affinity matured anti-HTRA1 VHH antibody blocking Elastase assay.
Figure 10 shows the results of hemolysis experiment induced by blocking the classical pathway of complement (Figure 10A) and hemolysis experiment induced by blocking the alternative pathway of complement (Figure 10B) with different combinations of anti-C5 VHH antibodies, as well as hemolysis experiment induced by blocking the classical pathway of complement (Figure 10C) and hemolysis experiment induced by blocking the alternative pathway of complement (Figure 10D) with anti-C5 antibodies with dual epitopes.
Figure 11 shows an exemplary structural schematic of a bispecific antibody.
Figure 12 shows the results of the hemolysis experiment induced by the bispecific antibody molecule blocking the complement classical pathway.
Figure 13 shows the results of the hemolysis test induced by the bispecific antibody molecule blocking the complement alternative pathway.
Figure 14 shows the results of the bispecific antibody molecule blocking H2-Opt assay.
Figure 15 shows the results of the bispecific antibody molecule blocking Elastase assay.
Figure 16 shows the results of an in vivo pharmacodynamic assay in which bispecific antibody molecules block sodium iodate-induced geographic atrophy.

### Detailed Description of Invention

### I. Definitions

It is to be understood that this invention is not limited to the particular methods, schemes, examples, and reagents described herein, as these may vary. It is also to be understood that the terminology used herein is for the purpose of describing particular embodiments only and is not intended to limit the scope of the invention, which is limited only by the appended claims.

Unless otherwise specified, all technical and scientific terms used herein have the same meanings as those generally understood by a person of ordinary skill in the art to which the present invention belongs.

For purposes of interpreting this specification, the following definitions will be used, and whenever appropriate, terms used in the singular may also include the plural and vice versa. It is to be understood that the terminology used herein is for the purpose of describing particular embodiments only and is not intended to be limiting.

The term "about" when used in conjunction with a numerical value is intended to encompass the numerical value within a range having a lower limit that is 5%, 4%, 3%, 2%, or 1% less than the numerical value specified, and an upper limit that is 5%, 4%, 3%, 2%, or 1% greater than the numerical value specified.

As used herein, the term "and/or" means any one of the options, or two or more, or all of the options.

When referring to "first" and "second" herein, the positions of the two domains are not indicated in any way, merely to distinguish the two domains or the two chains.

As used herein, the term "comprises" or "includes" means the inclusion of the stated element, integer, or step, but not the exclusion of any other element, integer, or step. When the term "comprises" or "includes" is used herein, it also encompasses a combination of the stated elements, integers, or steps unless otherwise indicated. For example, reference to an antibody variable region "comprising" a particular sequence is also intended to encompass antibody variable regions consisting of that particular sequence.

The term "HTRA1" as used herein refers to the serine protease HtrA1 serine peptidase 1 (UniProtKB/Swiss-Prot:Q92743). The terms "anti-HTRA1 antibody" and "an antibody that specifically binds to HTRA1" or "an antibody specifically binding HTRA1" refer to an antibody capable of binding HTRA1 with sufficient affinity. In an embodiment, the anti-HTRA1 antibodies of the invention are capable of specifically binding to HTRA1 protein or have high binding affinity for proteins expressing HTRA1. As used herein, the terms "C5" or "C5 protein" or "complement C5" are used interchangeably to refer to the complement component C5 protein of the complement system (UniProtKB/Swiss-Prot:P01031). The terms "anti-C5 antibody" and "an antibody specifically binding C5" or "an antibody specifically binding C5" refer to an antibody capable of binding C5 with sufficient affinity. In an embodiment, the anti-C5 antibody of the invention is capable of specifically binding a C5 protein or has a high binding affinity for a C5-expressing protein. In some aspects, the anti-C5 antibodies described herein also encompass anti-C5 antibodies that bind different epitopes. In some aspects, anti-C5 antibodies described herein also encompass bispecific or multispecific antibodies that bind C5 and other antigens. In some aspects, the anti-HTRA1 antibodies described herein also encompass bispecific or multispecific antibodies that bind HTRA1 and other antigens. In some aspects, the anti-C5 antibodies or anti-HTRA1 antibodies described herein also encompass bispecific antibodies that specifically bind both C5 and HTRA1.

General information on the nucleotide sequences of human immunoglobulin light and heavy chains is described in Kabat, E.A. et al., Sequences of Proteins of Immunological Interest, 5th Edition, Public Health Service, National Institutes of Health, Bethesda, MD (1991).

The bispecific antibodies of the invention may comprise linkers. The term "linker" as used herein refers to any molecule that enables direct attachment of different portions of a multispecific antibody. Examples of linkers that establish covalent links between different portions of the multispecific antibody include peptide linkers and non-protein polymers including, but not limited to, polyethylene glycol (PEG), polypropylene glycol, polyoxyalkylene, or copolymers of polyethylene glycol, polypropylene glycol. In some embodiments, the term "peptide linker" according to the invention refers to a sequence of amino acids wherein the sequence connects together the amino acid sequences of the various portions of the multispecific antibody. Preferably, the peptide linker is of a length sufficient to join two entities in such a way that they maintain their conformation with respect to each other so as not to interfere with the desired activity. Peptide linkers may or may not primarily include the following amino acid residues: Gly, Ser, Ala, or Thr. Useful linkers include glycine-serine polymers including, for example, (G)n (SEQ ID NO:38), (GS)n (SEQ ID NO:39), (GSGGS)n (SEQ ID NO:40), (GGGGS)n (SEQ ID NO:41), (GGGS)n (SEQ ID NO:42), and (GGGGS)nG (SEQ ID NO:43), wherein n is an integer of at least 1 (and preferably 2, 3, 4, 5, 6, 7, 8, 9, 10, 11, 12, 13, 14, or 15). Useful linkers also include glycine-alanine polymers, alanine-serine polymers, and other flexible linkers. Exemplary linkers include, for example, the sequence set forth in SEQ ID NO:26.

The term "Fc domain" or "Fc region" is used herein to define a C-terminus region of an immunoglobulin heavy chain that contains at least a portion of a constant region. This term includes native sequence Fc regions and variant Fc regions. A native immunoglobulin "Fc domain" comprises two or three constant domains, namely the CH2 domain, the CH3 domain, and the optional CH4 domain. For example, in native antibodies, the immunoglobulin Fc domain comprises the second and third constant domains (CH2 and CH3 domains) derived from the two heavy chains of IgG, IgA, and IgD class antibodies; or the second, third, and fourth constant domains (CH2, CH3, and CH4 domains) derived from the two heavy chains of antibodies of the IgM and IgE classes. Unless otherwise indicated herein, the amino acid residues in the Fc region or heavy chain constant region are numbered according to the EU numbering system (also referred to as the EU index) as described in Kabat et al., Sequences of Proteins of Immunological Interes, 5th Edition, Public Health Service, National Institutes of Health, Bethesda, MD, 1991. However, the C-terminus lysine (Lys447) of the Fc region may or may not be present. Two Fc regions can be dimerized to form a dimeric Fc, and two different Fcs can be heterodimerized to form a heterodimeric Fc. Herein, the terms "Fc region", "Fc portion" and "dimeric Fc (e.g., heterodimeric Fc)" do not include the heavy chain variable region VH and the light chain variable region VL and the heavy chain constant region CH1 and the light chain constant region CL of an immunoglobulin, but may include a hinge region at N-terminus of the heavy chain constant region in some cases. In an embodiment, a human IgG heavy chain Fc region is from Asp221 or from Cys226 or from Asp231 to the C-terminus of the heavy chain.

The term "vector" as used herein refers to a nucleic acid molecule capable of propagating another nucleic acid to which it is linked. The term includes vectors that are self-replicating nucleic acid structures, as well as vectors that are incorporated into the genome of a host cell into which they have been introduced. Some vectors are capable of directing the expression of a nucleic acid to which they are operably linked. Such vectors are referred to herein as "expression vectors".

The term "binding molecule" refers to any molecule capable of specifically binding to a target, such as an antibody or antigen-binding fragment or fusion protein thereof.

The terms "VHH" or "VHH antibody" are used interchangeably herein and generally refer to an antibody that comprises only one heavy chain variable region or consists thereof, has antigen binding activity. VHH typically comprises three CDRs and 4 highly conserved framework regions, and typically has the structure of the following formula: FR1-CDR1-FR2-CDR2-FR3-CDR3-FR4, where FR1 to FR4 refer to framework regions 1 to 4; CDR1 to CDR3 refer to complementarity determining regions 1-3. The CDR sequences in the VHH variable regions may be determined following any one of the CDR definition schemes described in the "Definitions" section, preferably the boundaries of the three CDRs in the variable region sequences may be defined by IMGT. VHHs typically include only heavy chain variable domains derived from heavy chain antibodies lacking the light chain, also known as nanobodies. The VHHs used in the present invention are preferably derived from camelids, such as alpacas, or humanized or sequence-optimized forms thereof (e.g., affinity matured forms to increase binding affinity). In some embodiments, the VHH of the invention is a monovalent monospecific polypeptide molecule consisting of or consisting essentially of a single heavy chain variable region (e.g. the heavy chain variable region of a heavy chain antibody).

The single domain antibodies or VHHs of the invention may also be comprised in larger polypeptides/proteins. Examples of polypeptides/proteins comprising a VHH of the invention include, but are not limited to, heavy chain antibodies (HcAb) or multispecific antibodies or fusion proteins.

"Heavy chain antibody" as used herein refers to an antibody that does not have a light chain, for example, it may comprise VH-Fc or VH-CH2-CH3 or VH-hinge region-CH2-CH3 from the N-terminus to the C-terminus, or may comprise VH-CH1-CH2-CH3. The heavy chain antibodies of the invention may also encompass homodimers, such as heavy chain dimer antibodies without light chains. The heavy chain antibody may comprise VH from a standard antibody or VH from a single domain antibody. For example, the VH in the heavy chain antibody may be VHH. In some embodiments, the heavy chain antibody of the invention can be a heavy chain antibody having a framework region and/or a heavy chain constant region derived from camelids (llamas, camels, especially alpacas), a humanized form thereof or a sequence-optimized form thereof (affinity matured form), or a fragment thereof (e.g., a fragment comprising at least a portion of the constant region). Heavy chain antibodies of the invention also encompass antibodies formed by fusing a heavy chain variable region or VHH to an Fc region (e.g., a human IgG Fc region, such as a human IgG1 or IgG4Fc region).

When referring to "VHH" in the context of a heavy chain antibody or multispecific antibody or fusion protein, it is to be understood that it is part of the multispecific antibody and not as a separate molecule.

The term "target" refers to the conjugated substance against which the binding molecule is directed. The target may be an antigen, a ligand, or a receptor. The term "antigen" refers to a molecule that elicits an immune response. Such an immune response may involve antibody production or activation of specific immune cells, or both. Those skilled will appreciate that any macromolecule, including substantially all proteins or peptides, may be used as an antigen. Furthermore, the antigen may be derived from recombinant or genomic DNA. As used herein, the term "epitope" refers to a portion of an antigen that specifically interacts with an antibody molecule. Where the binding molecules of the present invention refer to target-binding regions derived from antibodies, "target" and "antigen" may be used interchangeably.

The term "target-binding region" as used herein refers to the portion of a binding molecule, e.g., a multispecific binding molecule or a trispecific binding molecule, that binds to a particular target or antigen. The target-binding region may be, for example, an antibody or immunoglobulin per se or an antibody fragment. Such target-binding regions may or may not have tertiary structure independent of the remainder of the binding molecule and may or may not bind to their target as separate entities. The target-binding region may also be a receptor or a ligand, or a domain of a receptor capable of binding to a ligand. In the case of a multispecific antibody or bispecific antibody, a "target-binding region" is also referred to as an "antigen-binding region". The term "antigen-binding region" as used herein refers to any portion of an antibody or antigen-binding fragment thereof, e.g., a multispecific antibody or a bispecific antibody, that binds to a particular target or antigen. The antigen-binding region may be, for example, an antibody or immunoglobulin per se or an antibody fragment. Such antigen-binding regions may or may not have tertiary structure independent of the remainder of the multispecific or bispecific antibody, and may or may not bind to their antigen/epitope as separate entities. Where the binding molecules of the present invention refer to a target-binding region derived from an antibody, the terms "target-binding region" and "antigen-binding region" are used interchangeably. In an embodiment, the antigen-binding regions used for the multispecific antibody molecules of the invention may comprise only the VH, e.g., which is from VHH, e.g., which is VHH.

The term "multispecific binding molecule" refers to a multispecific binding molecule which is at least bispecific, e.g., a bispecific binding molecule, that is, a molecule comprising at least a first target-binding region and a second target-binding region, wherein the first target-binding region binds to one target or antigen and the second target-binding region binds to another antigen or target. The multispecific binding molecule according to the present invention also encompasses a multispecific molecule, such as bispecific binding molecule comprising multiple target-binding regions/binding sites. In some embodiments, the bispecific binding molecule of the present invention is a bispecific antibody. In some embodiments, the trispecific binding molecule of the present invention is a trispecific antibody. In some embodiments, the multispecific binding molecule of the invention is a fusion protein, which can, for example, comprise a target binding region from an antibody and a target binding region from a receptor or ligand.

The term "monospecific" as used herein refers to a polypeptide/protein molecule having one or more target binding regions, each site of which binds to the same site or structure of the same target or the same epitope of the same antigen. In some aspects, the monospecificity refers to binding of different target binding regions to the same antigen.

The term "multispecific" antibody as used herein refers to an antibody having at least two antigen-binding regions, each of which binds to a different antigen. A multispecific antibody is an antibody that has binding specificity for at least two different antigens. In an embodiment, provided herein is a bispecific antibody having binding specificity for a first antigen and a second antigen.

When referring to a "first antigen-binding region" in a multispecific or bispecific antibody, reference is made to a binding region that binds the first antigen without intent to limit the number of such antigen-binding regions contained in the antibody, e.g., one or more first antigen-binding regions may be included in the multispecific or bispecific antibody. For example, the bispecific antibody comprises a first antigen-binding region and a second antigen-binding region, but may comprise one or more of the first antigen-binding region and one or more of the second antigen-binding region. In some embodiments of the invention, the binding regions that bind the same antigen can be the same or different, e.g. the binding regions that bind the same antigen can bind different epitopes and thus be different. In an embodiment, the bispecific antibody molecules of the invention comprise two first antigen-binding regions that specifically bind C5 distinct epitopes and one second antigen-binding region that specifically binds HTRA1.

The term "variable region" or "variable domain" refers to a domain of an antibody heavy or light chain involved in the binding of the antibody to the antigen. The variable regions of the heavy and light chains of native antibodies generally have similar structures, with each domain containing four conserved framework regions (FRs) and three complementarity determining regions.

"Complementarity determining region" or "CDR region" or "CDR" is a region in an antibody variable domain that is highly variable in sequence and forms a structurally defined loop ("hypervariable loop") and/or comprises antigen-contacting residues ("antigen-contacting sites"). The CDRs are primarily responsible for the binding to antigenic epitopes. The CDRs of the heavy chain and light chain are commonly referred to as CDR1, CDR2, and CDR3, numbered sequentially from the N-terminus. The CDRs located in a heavy chain variable domain of an antibody are referred to as HCDR1, HCDR2 and HCDR3, whereas the CDRs located in a light chain variable domain of an antibody are referred to as LCDR1, LCDR2 and LCDR3. In a given amino acid sequence of light chain variable region or heavy chain variable region, the exact amino acid sequence boundaries of each CDR may be determined using any one or a combination of a number of well-known antibody CDR designated schemes, including, for example, Chothia scheme based on the three-dimensional structure of antibodies and the topology of CDR loops (Chothia et al., (1989) Nature 342: 877-883; Al-Lazikani et al., "Standard conformations for the canonical structures of immunoglobulins", Journal of Molecular Biology, 273, 927-948 (1997)); Kabat based on antibody sequence variability (Kabat et al., Sequences of Proteins of Immunological Interest, 4th ed., U.S. Department of Health and Human Services, National Institutes of Health (1987); AbM (University of Bath); Contact (University College London), International ImmunoGeneTics database (IMGT)(www.imgt.cines.fr/); and a North CDR definition based on affinity propagation clustering using a large number of crystal structures. Unless otherwise indicated, in the present invention, the term "CDR" or "CDR sequence" encompasses CDR sequences determined in any of the ways described above. CDRs may also be determined based on the same Kabat numbering position as a reference CDR sequence (e.g., any one of the exemplary CDRs in the present invention). In an embodiment, the HCDRs in the VHH antibodies of the invention may be defined in terms of either any one scheme or a combination of two or more schemes.

In an embodiment, the HCDRs in the VHH antibodies of the invention are defined as follows:
HCDR1 is defined by AbM scheme;
HCDR2 is defined by the Kabat scheme; and
HCDR3 is defined by AbM scheme.

A "humanized antibody" is an antibody that retains the antigen-specific reactivity of a non-human antibody(e.g., camlid original VHH antiboy), while being less immunogenic when administered to humans as a therapeutic drug. This can be achieved, for example, by retaining the non-human antigen-binding sites and replacing the remainder of the antibodies with their human counterparts (i.e., replacing the portions of the variable regions not involved in binding with the corresponding portions of a human antibody).

As used herein, the terms "anti," "binding," or "specific binding" mean that the binding to the target or antigen is selective and can be distinguished from unwanted or non-specific interactions. The ability of a binding site to bind to a particular target or antigen can be determined by flow cytometry, enzyme-linked immunosorbent assay (ELISA), or conventional binding assays known in the art, such as by radioimmunoassay (RIA), bio-layer interferometry, MSD assay, or surface plasmon resonance (SPR).

"Affinity" or "binding affinity" refers to the inherent binding affinity that reflects the interaction between members of a binding pair. The affinity of a molecule X for its partner Y can be generally represented by the dissociation constant (K_{D}), which is the ratio of the dissociation and association rate constants (K_{dis} and Kₒₙ, respectively). The affinity can be measured by common methods known in the art. One particular method for measuring the affinity is the ForteBio kinetic binding assay herein.

The term "host cell" refers to a cell into which an exogenous polynucleotide has been introduced, including the progeny of such cells. Host cells include "transformants" and "transformed cells," which include primary transformed cells and progeny derived therefrom.

The terms "individual" or "subject" are used interchangeably herein to refer to mammals. Mammals include, but are not limited to, domesticated animals (e.g., cows, sheep, cats, dogs, and horses), primates (e.g., humans and non-human primates such as monkeys), rabbits, and rodents (e.g., mice and rats). In particular, the individual is a human.

The term "treating" (or "treat" or "treatment") refers to slowing, interrupting, arresting, relieving, stopping, lowering, or reversing the onset of the symptoms, complications, or biochemical indicators of a disease, relieving the symptoms, or preventing or inhibiting the further development of the disease, condition, or disorder.

The term "preventing" includes the inhibition of the occurrence or development of a disease or condition or the symptoms of a particular disease or condition.

The term "therapeutic agent" as used herein encompasses any active substance or active agent that is effective in the prevention or treatment of ocular diseases or conditions, for example in the treatment of ocular diseases.

The term "combination therapy" refers to the administration of two or more therapeutic agents or treatment modalities to treat the diseases described herein. Such administration includes co-administering the therapeutic agents in a substantially simultaneous manner, for example, in a single capsule having a fixed ratio of active ingredients. Alternatively, such administration includes co-administration of the active ingredients in multiple or separate containers (e.g., tablets, capsules, powders, and liquids). The powder and/or liquid may be reconstituted or diluted to the desired dosage prior to administration. In addition, such administration also includes the use of each type of therapeutic agent at approximately the same time or at different times in a sequential manner. In either case, the treatment regimen will provide a beneficial effect of the pharmaceutical combination in treating the disorders or conditions described herein.

The term "pharmaceutical combination" or "combination product" refers to a non-fixed combination product or a fixed combination product, comprising but not limited to kit, pharmaceutical composition. In some embodiments, the pharmaceutical composition is contained in a kit. The term "non-fixed combination" means that the active ingredients (e.g., (i) the of antibody of the present invention, and (ii) an additional therapeutic agent) are administered, either simultaneously, without specific time limitation or sequentially at identical or different time intervals), to a patient as separate entities, wherein such administration provides two or more prophylactically or therapeutically effective active agents in the patient. The term "fixed combination" means that two or more active agents are administered to a patient simultaneously in the form of a single entity. The dosage and/or time intervals of the two or more active agents are preferably selected such that the combined use of the components results in a greater effect in the treatment of the disease or condition than that would be achieved by use of any component alone. The ingredients may each be in separate formulations, which may be the same or different.

The term "pharmaceutical composition" refers to a composition that is present in a form that allows the biological activity of the active ingredients contained therein to be effective, and which does not contain additional ingredients having unacceptable toxicity to the subject to which the composition is administered.

The term "pharmaceutically acceptable auxiliary material" refers to diluents, adjuvants (e.g., Freund's adjuvants (complete and incomplete)), excipients, carriers, or stabilizers, etc., with which the active substances are administered.

A "subject/patient/individual sample" refers to a collection of cells or fluids obtained from a patient or subject. The source of the tissue or cell sample may be a solid tissue, such as from fresh, frozen, and/or preserved organs or tissue samples, biopsy samples, or puncture samples; body fluids such as tears, vitreous fluid, cerebrospinal fluid, amnion fluid (amniotic fluid), peritoneal fluid (ascites fluid), or interstitial fluid.

### II. Anti-C5 antibody

An aspect of the invention provides a C5 antibody having a higher binding affinity for C5. In some embodiments, the C5 antibodies of the invention are suitable for use in construction of the antigen-binding regions in the multispecific antibody molecules of the invention.

In some embodiments, the anti-C5 antibody of the invention binds C5 (e.g. human C5 or cynomolgus monkey C5) with greater affinity. In some embodiments, the anti-C5 antibody of the invention is capable of binding human C5 and/or cynomolgus monkey C5 with high affinity, for example with a K _{D} value of less than or equal to about 2 nM, for example less than or equal to about 5, 4, 3, 2, 1.5, 1, 0.5, 0.4, 0.3, 0.25, 0.2 or 0.15 nM. In some embodiments, the anti-C5 antibodies of the invention bind human C5 and/or cynomolgus monkey C5 with a K _{D} value greater than about 0.05 nM or about 0.1 nM. In some embodiments, the anti-C5 antibody of the invention binds human C5 and/or cynomolgus monkey C5 with a K _{D} value between any two of the numerical ranges described above. In some embodiments, the binding affinity of the anti-C5 antibody of the invention is determined by bio-layer interferometry.

In some embodiments, the anti-C5 antibody of the invention is capable of blocking the complement classical pathway, for example, blocking hemolysis induced thereby.

In some embodiments, the anti-C5 antibody of the invention is capable of blocking the complement alternative pathway, for example, blocking hemolysis induced thereby.

In some embodiments, the anti-C5 antibody of the invention is capable of blocking the interaction of C5 with C5 convertase, blocking C5a thus formed, e.g., blocking production of C5a in the complement classical pathway.

In some embodiments, the anti-C5 antibody of the invention is capable of completely inhibiting the biological activity of C5.

In some embodiments, the anti-C5 antibody of the invention is a single domain antibody, particularly a VHH antibody.

In some embodiments, the anti-C5 antibody of the invention is a VHH-His antibody, e.g. a VHH-6His antibody, bearing a His-tag (e.g. 6-His) at the N-terminus or the C-terminus.

In some embodiments, the anti-C5 single domain antibodies of the invention are VHH antibodies comprising or consisting of a heavy chain variable region typically having the following structure: FR1-VHH CDR1-FR2-VHH CDR2-FR3-VHH CDR3-FR4, wherein FR1-FR4 refer to framework regions 1-4; VHH CDR1 to VHH CDR3 refer to complementarity determining regions 1-3. The CDR sequences in the VHH variable regions may be determined following any one of the CDR definition schemes described in the "Definitions" section, preferably the boundaries of the three CDRs in the VHH sequences may be defined as follows: CDR1 defined according to AbM, CDR2 defined according to Kabat, and CDR3 defined by AbM.

In some embodiments, the anti-C5 VHH antibodies of the invention comprise
(i) three complementarity determining regions (CDRs) contained in the VH set froth in any one of SEQ ID NOs: 17-23, or
(ii) a sequence comprising a total of at least one and no more than 5, 4, 3, 2, or 1 amino acid changes (preferably amino acid substitutions, preferably conservative substitutions) on the three CDR regions relative to the sequence of (i);
   preferably, the CDR1 sequence is defined according to AbM, CDR2 is defined according to Kabat, and CDR3 is defined according to AbM.

In some embodiments, the anti-C5 VHH antibodies of the invention comprise or consist of a heavy chain variable region, which comprises
(i) three complementarity determining regions (CDRs) contained in the VH of any one of SEQ ID NOs: 17-23, or
(ii) a sequence comprising a total of at least one and no more than 5, 4, 3, 2, or 1 amino acid changes (preferably amino acid substitutions, preferably conservative substitutions) on the three CDR regions relative to the sequence of (i);
preferably, the CDR1 sequence is defined according to AbM, the CDR2 sequence is defined according to Kabat, and the CDR3 sequence is defined according to AbM.

In some embodiments, the anti-C5 VHH antibodies of the invention comprise complementarity determining regions (CDRs) VHH CDR1, VHH CDR2 and VHH CDR3. In some embodiments, the anti-C5 VHH of the invention comprises or consists of a heavy chain variable region comprising complementarity determining regions (CDRs) VHH CDR1, VHH CDR2 and VHH CDR3.

In some embodiments, the VHH CDR1 comprises or consists of an amino acid sequence selected from the group consisting of SEQ ID NO: 1 or 4, or the VHH CDR1 comprises an amino acid sequence with one, two or three changes (preferably amino acid substitutions, preferably conservative substitutions) compared to the amino acid sequence selected from the group consisting of SEQ ID NO: 1 or 4.

In some embodiments, the VHH CDR2 comprises or consists of the amino acid sequence of SEQ ID NOs: 2, 5, 7, 8 or 9, or the VHH CDR2 comprises an amino acid sequence with one, two or three changes (preferably amino acid substitutions, preferably conservative substitutions) compared to the amino acid sequence of SEQ ID NOs: 2, 5, 7, 8 or 9.

In some embodiments, the VHH CDR3 comprises or consists of an amino acid sequence selected from the group consisting of SEQ ID NOs: 3, 6, 10 or 30, or the VHH CDR3 comprises an amino acid sequence with one, two or three changes (preferably amino acid substitutions, preferably conservative substitutions) compared to the amino acid sequence selected from the group consisting of SEQ ID NOs: 3, 6, 10 or 30.

In an embodiment, the anti-C5 VHH antibody of the invention comprises complementarity determining regions (CDRs) VHH CDR1, VHH CDR2 and VHH CDR3, wherein
(i) VHH CDR1 comprises or consists of the amino acid sequence set forth in SEQ ID NO:4, VHH CDR2 comprises or consists of the amino acid sequence set forth in SEQ ID NOs:5 or 7 or 8, VHH CDR3 comprises or consists of the amino acid sequence set forth in SEQ ID NOs:6 or 30; or
(ii) VHH CDR1 comprises or consists of the amino acid sequence set forth in SEQ ID NO:4, VHH CDR2 comprises or consists of the amino acid sequence set forth in SEQ ID NO:5, VHH CDR3 comprises or consists of the amino acid sequence set forth in SEQ ID NO:6 or 30; or
(iii) VHH CDR1 comprises or consists of the amino acid sequence set forth in SEQ ID NO:4, VHH CDR2 comprises or consists of the amino acid sequence set forth in SEQ ID NOs: 7 or 8, VHH CDR3 comprises or consists of the amino acid sequence set forth in SEQ ID NO:6; or
(iv) VHH CDR1 comprises or consists of the amino acid sequence set forth in SEQ ID NO:1, VHH CDR2 comprises or consists of the amino acid sequence set forth in SEQ ID NO:2 or 9, VHH CDR3 comprises or consists of the amino acid sequence set forth in SEQ ID NO:3 or 10; or
(v) VHH CDR1 comprises or consists of the amino acid sequence set forth in SEQ ID NO:1, VHH CDR2 comprises or consists of the amino acid sequence set forth in SEQ ID NO:2, VHH CDR3 comprises or consists of the amino acid sequence set forth in SEQ ID NO:3; or
(vi) VHH CDR1 comprises or consists of the amino acid sequence set forth in SEQ ID NO:1, VHH CDR2 comprises or consists of the amino acid sequence set forth in SEQ ID NO:9, VHH CDR3 comprises or consists of the amino acid sequence set forth in SEQ ID NO:10.

In an embodiment, the anti-C5 VHH antibody of the invention comprises or consists of a heavy chain variable region, wherein said heavy chain variable region comprises the complementarity determining regions (CDRs) VHHCDR1, VHHCDR2 and VHHCDR3, wherein
(i) VHH CDR1 comprises or consists of the amino acid sequence set forth in SEQ ID NO:4, VHH CDR2 comprises or consists of the amino acid sequence set forth in SEQ ID NO:5 or 7 or 8, VHH CDR3 comprises or consists of the amino acid sequence set forth in SEQ ID NO:6 or 30; or
(ii) VHH CDR1 comprises or consists of the amino acid sequence set forth in SEQ ID NO:4, VHH CDR2 comprises or consists of the amino acid sequence set forth in SEQ ID NO:5, VHH CDR3 comprises or consists of the amino acid sequence set forth in SEQ ID NO:6 or 30; or
(iii) VHH CDR1 comprises or consists of the amino acid sequence set forth in SEQ ID NO:4, VHH CDR2 comprises or consists of the amino acid sequence set forth in SEQ ID NOs: 7 or 8, VHH CDR3 comprises or consists of the amino acid sequence set forth in SEQ ID NO:6; or
(iv) VHH CDR1 comprises or consists of the amino acid sequence set forth in SEQ ID NO:1, VHH CDR2 comprises or consists of the amino acid sequence set forth in SEQ ID NO:2 or 9, VHH CDR3 comprises or consists of the amino acid sequence set forth in SEQ ID NO:3 or 10; or
(v) VHH CDR1 comprises or consists of the amino acid sequence set forth in SEQ ID NO:1, VHH CDR2 comprises or consists of the amino acid sequence set forth in SEQ ID NO:2, VHH CDR3 comprises or consists of the amino acid sequence set forth in SEQ ID NO:3; or
(vi) VHH CDR1 comprises or consists of the amino acid sequence set forth in SEQ ID NO:1, VHH CDR2 comprises or consists of the amino acid sequence set forth in SEQ ID NO:9, VHH CDR3 comprises or consists of the amino acid sequence set forth in SEQ ID NO:10.

In some embodiments, the anti-C5 VHH antibodies of the invention comprise or consist of a heavy chain variable region, which
(i) comprises or consists of an amino acid sequence having at least 90%, 91%, 92%, 93%, 94%, 95%, 96%, 97%, 98% or 99% identity to an amino acid sequence selected from any one set forth in SEQ ID NOs: 17-23; or
(ii) comprises or consists of an amino acid sequence selected from any one set forth in SEQ ID NOs: 17-23; or
(iii) an amino acid sequence having one or more (preferably no more than 10, more preferably no more than 5, 4, 3, 2, 1) amino acid changes (preferably amino acid substitutions, more preferably conservative amino acid substitutions) compared to an amino acid sequence selected from the group consisting of any one of SEQ ID NOs: 17-23, preferably the amino acid changes do not occur in the CDR regions.

In some embodiments, the anti-C5 VHH antibody of the invention comprises or consists of an amino acid sequence selected from any one set forth in SEQ ID NOs: 17-23.

In an embodiment, the anti-C5 VHH antibodies of the invention are humanized antibodies. Humanization may be achieved by substituting one or more amino acid residues, particularly framework region sequences, in native VHH sequences of non-human origin (e.g., VHH sequences from alpacas or alpaca immunization) with residues at positions corresponding to the heavy chain VH from human conventional antibodies. Methods of humanizing VHH are well known in the art, such as those described in Example 3. In general, humanization substitutions are made in a manner that maintains the favorable binding properties of single domain antibodies. Assays for determining the biological properties, e.g., binding affinity, etc., of humanized single domain antibodies to identify and select appropriate mutations or combinations of mutations of humanized residues are well known in the art.

In some embodiments, the humanized single domain antibody of the invention may be obtained by a method comprising the steps of:
CDR loop structures were determined for parent single domain antibodies (e.g., camlid-derived VHH antibodies from phage display library screening):
**(1).** Determining CDR loop structure;
**(2).** Finding the closest homologous sequence for each V/J region of VHH in a human germline sequence database;
**(3).** Constructing the CDR region of VHH on the human framework region;
**(4).** Using sequence and structural features to identify amino acid positions in the framework region that serve to maintain CDR functions;
**(5).** Carrying out reverse mutations at sequence positions determined to be important;
**(6).** Optimizing amino acids at risk positions.

In an aspect of the invention, the invention also provides an anti-C5 antibody comprising two or more VHHs of the invention, for example VHH antibodies in tandem, wherein different VHH antibodies bind different epitopes on C5, for example linked in tandem with or without a linker.

In an embodiment, the different VHHs are linked by N- and N-termini, C- and C-termini, or N- and C-termini.

In an embodiment, the anti-C5 antibody of the invention comprises two or three VHHs binding different epitopes linked in tandem via linkers.

In some embodiments, the anti-C5 antibody of the invention comprises or consists of a chain comprising from the N-terminus to the C-terminus:
(1) the VHH1 specifically binding C5 described herein - the VHH2 specifically binding C5 described herein,
(2) the VHH1 specifically binding C5 described herein - the VHH1 specifically binding C5 described herein - the VHH2 specifically binding C5 described herein, or
(3) the VHH1 specifically binding C5 described herein - the VHH2 specifically binding C5 described herein - the VHH1 specifically binding C5 described herein,
optionally, the VHHs are linked by a linker.

In some embodiments, the VHH1 specifically binding C5 and the VHH2 specifically binding C5 are anti-C5 VHHs described herein binding different epitopes. In some embodiments, the VHH1 that specifically binds C5
(i) comprises a complementarity determining region (CDR) VHH CDR1, a VHH CDR2 and a VHH CDR3, wherein the VHH CDR1 comprises or consists of the amino acid sequence set forth in SEQ ID NO:4, the VHH CDR2 comprises or consists of the amino acid sequence set forth in SEQ ID NO:5 or 7 or 8, the VHH CDR3 comprises or consists of the amino acid sequence set forth in SEQ ID NO:6 or 30;
   VHH CDR1 comprises or consists of the amino acid sequence set forth in SEQ ID NO:4, VHH CDR2 comprises or consists of the amino acid sequence set forth in SEQ ID NO:5, VHH CDR3 comprises or consists of the amino acid sequence set forth in SEQ ID NO:6 or 30; or
   VHH CDR1 comprises or consists of the amino acid sequence set forth in SEQ ID NO:4, VHH CDR2 comprises or consists of the amino acid sequence set forth in SEQ ID NOs: 7 or 8, VHH CDR3 comprises or consists of the amino acid sequence set forth in SEQ ID NO:6;
      or
(ii) comprises a heavy chain variable region comprising or consisting of an amino acid sequence set forth in any one of SEQ ID NOs: 18-21, or an amino acid sequence having at least 90% identity thereto;
and VHH2 specifically binding C5
(i) comprises complementarity determining regions (CDRs) VHH CDR1, VHH CDR2 and VHH CDR3, wherein
   (a) VHH CDR1 comprises or consists of the amino acid sequence set forth in SEQ ID NO:1, VHH CDR2 comprises or consists of the amino acid sequence set forth in SEQ ID NO:2 or 9, VHH CDR3 comprises or consists of the amino acid sequence set forth in SEQ ID NO:3 or 10; or
   (b) VHH CDR1 comprises or consists of the amino acid sequence set forth in SEQ ID NO:1, VHH CDR2 comprises or consists of the amino acid sequence set forth in SEQ ID NO:2, VHH CDR3 comprises or consists of the amino acid sequence set forth in SEQ ID NO:3; or
   (c) VHH CDR1 comprises or consists of the amino acid sequence set forth in SEQ ID NO:1, VHH CDR2 comprises or consists of the amino acid sequence set forth in SEQ ID NO:9, and VHH CDR3 comprises or consists of the amino acid sequence set forth in SEQ ID NO:10;
   (ii) comprises a heavy chain variable region comprising or consisting of an amino acid sequence set forth in any one of SEQ ID NOs: 17, 22, or 23, or an amino acid sequence having at least 90% identity thereto;
      or vice versa.

In some embodiments, the VHH1 specifically binding C5 comprises or consists of an amino acid sequence as set forth in SEQ ID NO:20. In some embodiments, the VHH2 specifically binding C5 comprises or consists of an amino acid sequence as set forth in SEQ ID NO:23.

In some embodiments, the linker comprises or consists of the amino acid sequence set forth in SEQ ID NO: 26.

In some embodiments, the anti-C5 antibody of the invention comprises a chain comprising or consisting of the amino acid sequence set forth in SEQ ID NOs: 34, 35 or 36, or an amino acid sequence having at least 90%, 91%, 92%, 93%, 94%, 95%, 96%, 97%, 98% or 99% identity thereto.

In some embodiments, the anti-C5 antibody of the invention comprises or consists of the amino acid sequence set forth in SEQ ID NOs: 34, 35 or 36, or an amino acid sequence having at least 90%, 91%, 92%, 93%, 94%, 95%, 96%, 97%, 98% or 99% identity thereto.

### III. Anti-HTRA1 antibody

An aspect of the invention provides an HTRA1 antibody having a higher binding affinity to HTRA1. In some embodiments, the HTRA1 antibodies of the invention are suitable for use in the construction of antigen-binding regions in multispecific antibody molecules.

In some embodiments, the anti-HTRA1 antibodies of the invention bind to HTRA1 (e.g. human HTRA1 or cynomolgus monkey HTRA1) with higher affinity. In some embodiments, the anti-HTRA1 antibody of the invention is capable of binding human HTRA1 and/or cynomolgus monkey HTRA1 with high affinity, for example with a K_{D} value of less than or equal to about 3 nM, for example less than or equal to about 5, 4, 3, 2, 1 or 0.9 nM. In some embodiments, the anti-HTRA1 antibodies of the invention bind human HTRA1 and/or cynomolgus monkey HTRA1 with a K _{D} value greater than about 0.1 nM or about 0.5 nM. In some embodiments, the anti-HTRA1 antibodies of the invention bind to human HTRA1 and/or cynomolgus monkey HTRA1 with a K _{D} value between any two numerical ranges described above. In some embodiments, the binding affinity of the anti-C5 antibody of the invention is determined by bio-layer interferometry.

In some embodiments, the anti-HTRA1 antibody of the invention is capable of inhibiting activity of HTRA1, for example, inhibiting activity of its serine protease or elastase. In some embodiments, the anti-HTRA1 antibody of the invention is capable of blocking activity of HTRA1 on an enzymatic cleavage of H2-Opt. In some embodiments, the anti-HTRA1 antibody of the invention is capable of blocking activity of HTRA1 on an enzymatic cleavage of elastin.

In some embodiments, the anti-HTRA1 antibody of the invention is a single domain antibody, in particular a VHH antibody.

In some embodiments, the anti-HTRA1 single domain antibody of the invention is a VHH antibody comprising or consisting of a heavy chain variable region, typically having the following structure: FR1-VHH-CDR1-FR2-VHH CDR2-FR3-VHH CDR3-FR4, wherein FR1-FR4 refer to framework regions 1-4; VHH CDR1 to VHH CDR3 refer to complementarity determining regions 1-3. The CDR sequences in the VHH variable regions may be determined following any one of the CDR definition schemes described in the "Definitions" section, preferably the boundaries of the three CDRs in the VHH sequences may be defined by IMGT.

In some embodiments, the anti-HTRA1 VHH antibody of the invention comprises
(i) three complementarity determining regions (CDRs) contained in the VH set forth in SEQ ID NOs: 24 or 25, or
(ii) a sequence comprising a total of at least one and no more than 5, 4, 3, 2, or 1 amino acid changes (preferably amino acid substitutions, preferably conservative substitutions) on the three CDR regions relative to the sequence of (i);
preferably, the CDR1 sequence is defined according to AbM, CDR2 is defined according to Kabat, and CDR3 is defined according to AbM.

In some embodiments, the anti-HTRA1 VHH antibody of the invention comprises or consists of a heavy chain variable region comprising
(i) three complementarity determining regions (CDRs) contained in the VH set forth in SEQ ID NOs: 24 or 25, or
(ii) a sequence comprising a total of at least one and no more than 5, 4, 3, 2, or 1 amino acid changes (preferably amino acid substitutions, preferably conservative substitutions) on the three CDR regions relative to the sequence of (i);
preferably, the CDR1 sequence is defined according to AbM, CDR2 is defined according to Kabat, and CDR3 is defined according to AbM.

In some embodiments, the anti-HTRA1 antibody of the invention comprises complementarity determining regions (CDRs) VHH CDR1, VHH CDR2 and VHH CDR3. In some embodiments, the anti-HTRA1 VHH of the invention comprises or consists of a heavy chain variable region comprising a complementarity determining region (CDR) VHH CDR1, VHH CDR2 and VHH CDR3.

In some embodiments, the VHH CDR1 comprises or consists of amino acid sequence set forth in SEQ ID NO: 11 or 14, or the VHH CDR1 comprises an amino acid sequence with one, two or three changes (preferably amino acid substitutions, preferably conservative substitutions) compared to the amino acid sequence set forth in SEQ ID NOs: 11 or 14.

In some embodiments, the VHH CDR2 comprises or consists of the amino acid sequence set forth in SEQ ID NOs: 12 or 15, or the VHH CDR2 comprises an amino acid sequence with one, two or three changes (preferably amino acid substitutions, preferably conservative substitutions) compared to the amino acid sequence set forth in SEQ ID NOs: 12 or 15.

In some embodiments, the VHH CDR3 comprises or consists of amino acid sequence set forth in SEQ ID NOs: 13 or 16, or the VHH CDR3 comprises an amino acid sequence with one, two or three changes (preferably amino acid substitutions, preferably conservative substitutions) compared to the amino acid sequence set forth in SEQ ID NOs: 13 or 16.

In an embodiment, the anti-HTRA1 antibody of the invention comprises complementarity determining regions (CDRs) VHH CDR1, VHH CDR2 and VHH CDR3, wherein
(i) VHH CDR1 comprises or consists of the amino acid sequence set forth in SEQ ID NO:11, VHH CDR2 comprises or consists of the amino acid sequence set forth in SEQ ID NO:12, VHH CDR3 comprises or consists of the amino acid sequence set forth in SEQ ID NO:13; or
(ii) VHH CDR1 comprises or consists of the amino acid sequence set forth in SEQ ID NO:14, VHH CDR2 comprises or consists of the amino acid sequence set forth in SEQ ID NO:15, VHH CDR3 comprises or consists of the amino acid sequence set forth in SEQ ID NO:16; or
(iii) VHH CDR1 comprises or consists of the amino acid sequence set forth in SEQ ID NO:11 or 14, VHH CDR2 comprises or consists of the amino acid sequence set forth in SEQ ID NO:12 or 15, VHH CDR3 comprises or consists of the amino acid sequence set forth in SEQ ID NO:13 or 16.

In an embodiment, an anti-HTRA1 VHH antibody of the invention comprises or consists of a heavy chain variable region, wherein said heavy chain variable region comprises the complementarity determining regions (CDRs) VHHCDR1, VHHCDR2 and VHHCDR3, wherein
(i) VHH CDR1 comprises or consists of the amino acid sequence set forth in SEQ ID NO:11, VHH CDR2 comprises or consists of the amino acid sequence set forth in SEQ ID NO:12, VHH CDR3 comprises or consists of the amino acid sequence set forth in SEQ ID NO:13; or
(ii) VHH CDR1 comprises or consists of the amino acid sequence set forth in SEQ ID NO:14, VHH CDR2 comprises or consists of the amino acid sequence set forth in SEQ ID NO:15, VHH CDR3 comprises or consists of the amino acid sequence set forth in SEQ ID NO:16;
(iii) VHH CDR1 comprises or consists of the amino acid sequence set forth in SEQ ID NO:11 or 14, VHH CDR2 comprises or consists of the amino acid sequence set forth in SEQ ID NO:12 or 15, VHH CDR3 comprises or consists of the amino acid sequence set forth in SEQ ID NO:13 or 16.

In some embodiments, the anti-HTRA1 antibody of the invention comprises or consists of a heavy chain variable region, which
(i) comprises or consists of an amino acid sequence having at least 90%, 91%, 92%, 93%, 94%, 95%, 96%, 97%, 98% or 99% identity to amino acid sequence set forth in SEQ ID NO: 24 or 25; or
(ii) comprises or consists of amino acid sequence set forth in SEQ ID NO: 24 or 25; or
(iii) comprises an amino acid sequence with one or more (preferably no more than 10, more preferably no more than 5, 4, 3, 2, 1) amino acid changes (preferably amino acid substitutions, more preferably conservative amino acid substitutions) compared to amino acid sequence selected from any one set forth in SEQ ID NO: 24 or 25, preferably the amino acid changes do not occur in the CDR regions.

In some embodiments, the anti-HTRA1 VHH antibody of the invention comprises or consists of the amino acid sequence selected from any one set forth in SEQ ID NO: 24 or 25.

In an embodiment, the anti-HTRA1 VHH antibodies of the invention are humanized antibodies. Humanization may be achieved by substituting one or more amino acid residues, particularly framework region sequences, in native VHH sequences of non-human origin (e.g., VHH sequences from alpacas or alpaca immunization) with residues at positions corresponding to the heavy chain VH from human conventional antibodies. Methods of humanizing VHH are well known in the art, such as those described in Example 3. In general, humanization substitutions are made in a manner that maintains the favorable binding properties of single domain antibodies. Assays for determining the biological properties, e.g., binding affinity, etc., of humanized single domain antibodies to identify and select appropriate mutations or combinations of mutations of humanized residues are well known in the art.

In some embodiments, the humanized single domain antibody of the invention may be obtained by a method comprising the steps of:
CDR loop structures were determined for parent single domain antibodies (e.g., camlid-derived VHH antibodies from phage display library screening):
(1). Determining CDR loop structure;
(2). Finding the closest homologous sequence for each V/J region of VHH in a human germline sequence database;
(3). Constructing the CDR region of VHH on the human framework region;
(4). Using sequence and structural features to identify amino acid positions in the framework region that serve to maintain CDR functions;
(5). Carrying out reverse mutations at sequence positions determined to be important;
(6). Optimizing amino acids at risk positions.

### IV. Anti-C5 heavy chain antibody or anti-HTRA1 heavy chain antibody

In another aspect of the invention, the invention also provides a heavy chain antibody comprising a VHH antibody heavy chain variable region of the invention.

In some embodiments, a single domain antibody or a VHH of the invention (e.g., camlid-derived VHH or a humanized form thereof) may be linked to a constant region of a human antibody or a portion thereof, e.g., an Fc region, to generate a heavy chain antibody comprising a VHH-constant region or VHH-CH1-Fc or VHH-Fc. In an embodiment, the heavy chain antibody comprises a VHH antibody of the invention and an Fc region located at its C-terminus. In some embodiments, the VHH is linked to the Fc by a hinge region or a portion thereof, for example from a hinge region of IgG (e.g. a hinge region of IgG1, 2, 3, or 4) or a portion thereof.

In an aspect of the invention, the anti-C5 antibody of the invention is an anti-C5 heavy chain antibody. In some embodiments, the anti-C5 heavy chain antibody of the invention comprises an anti-C5 VHH as defined herein or a heavy chain variable region therein, and a heavy chain constant region or an Fc region of the heavy chain constant region.

In an aspect of the invention, the anti-HTRA1 antibody of the invention is an anti-HTRA1 heavy chain antibody. In some embodiments, the anti-HTRA1 heavy chain antibody of the invention comprises an anti-HTRA1 VHH as defined herein or a heavy chain variable region therein, and a heavy chain constant region or an Fc region of the heavy chain constant region.

In some embodiments, the heavy chain antibody comprises a constant region from a human or non-human primate (e.g., cynomolgus monkey) antibody, for example a constant region from human IgG1, human IgG2, human IgG3, or human IgG4.

In some embodiments, the heavy chain antibody comprises an Fc portion from human or non-human primate (e.g., cynomolgus monkey). In still another embodiment, the heavy chain antibody comprises a human IgG Fc region, for example a human IgG1, human IgG2, human IgG3, or human IgG4 Fc region, preferably a human IgG1 or human IgG4Fc region.

In an embodiment, the heavy chain antibody according to the invention may dimerize with another polypeptide chain comprising the Fc region (e.g., the same or different another heavy chain antibody) via the Fc region. Thus, in an embodiment, the invention also provides homologous or heterologous multimeric proteins comprising a heavy chain antibody of the invention. In a preferred embodiment, preferably the protein comprises a heavy chain antibody formed by pairing two identical heavy chain antibody chains.

In some embodiments, the anti-C5 antibody of the invention comprises a heavy chain comprising a heavy chain variable region and an Fc region. In some embodiments, the anti-C5 antibody of the invention comprises or consists of a heavy chain comprising or consisting of the heavy chain variable region of the anti-C5 VHH of the invention and Fc region.

In some embodiments, the anti-HTRA1 antibody of the invention comprises a heavy chain comprising a heavy chain variable region and an Fc region. In some embodiments, the anti-HTRA1 antibody of the invention comprises or consists of a heavy chain comprising or consisting of the heavy chain variable region of the anti-HTRA1 VHH of the invention and Fc region.

### V. Multi-specific antibody

In some embodiments, the invention provides a multispecific antibody, e.g. a bispecific antibody, that specifically binds C5, and optionally other antigens, e.g. HTRA1. In some embodiments the described multispecific antibodies comprise an antigen-binding region specifically binding C5 (for example the anti-C5 VHH antibodies or heavy chain antibodies of the invention), for example one or more antigen-binding regions which specifically bind C5, and optionally other antigen-binding regions, for example an antigen-binding region specifically binding HTRA1.

In some embodiments, the invention provides a multispecific antibody, e.g. a bispecific antibody, that specifically binds HTRA1, and optionally other antigens such as C5. In some embodiments the described multispecific antibodies comprise an antigen-binding region specifically binding HTRA1 (for example the anti-HTRA1 VHH antibody or the heavy chain antibody of the invention), for example one or more antigen-binding regions which specifically bind HTRA1 and, optionally, other antigen-binding regions, for example an antigen-binding region specifically binding C5.

In some embodiments, the invention provides a multispecific antibody, e.g. a bispecific antibody, that specifically binds C5 and HTRA1, and optionally other antigens.

Thus, one aspect of the invention is directed to a multispecific antibody, such as a bispecific antibody comprising at least 2 different antigen-binding regions specifically binding C5 and an antigen-binding region specifically binding HTRA1.

In some embodiments, the antigen-binding region specifically binding C5 is from an anti-C5 antibody, for example from an anti-C5 antibody described herein, for example an anti-C5 VHH described herein. In some embodiments, the two different antigen-binding regions specifically binding C5 bind different C5 epitopes. In some embodiments, the two different antigen-binding regions specifically binding C5 are two different anti-C5 VHHs described herein.

In some embodiments, the antigen-binding region specifically binding HTRA1 is from an anti-HTRA1 antibody, for example from an anti-HTRA1 antibody described herein, for example an anti-HTRA1 VHH described herein.

In some embodiments, the bispecific antibodies of the invention may comprise one or more antigen-binding regions specifically binding HTRA1. In an embodiment, the bispecific antibody comprises one antigen-binding region specifically binding HTRA1. In an embodiment, the bispecific antibody comprises 2 antigen-binding regions specifically binding C5, which binds different C5 epitopes and one antigen-binding region specifically binding HTRA1.

In some embodiments, the bispecific antibodies of the invention may comprise 2 different anti-C5 VHHs and one anti-HTRA1 VHH, with 3 VHHs linked in tandem. In some embodiments, the VHHs of the bispecific antibodies of the invention are linked via a linker or not via a linker. In some embodiments, the VHHs of the bispecific antibodies of the invention are all linked by a linker.

In some embodiments, in the bispecific antibodies of the invention, the N-terminus of one VHH is linked to the C-terminus of another VHH, or the N-terminus of one VHH is linked to the N-terminus of another VHH, or the C-terminus of one VHH is linked to the C-terminus of another VHH.

In some embodiments, the bispecific antibodies of the invention comprise or consist of a chain comprising from the N-terminus to the C-terminus:
VHH1 specifically binding C5-VHH2 specifically binding C5-VHH specifically binding HTRA1,
the VHHs are linked with or without a linker.
In some embodiments, the VHH1 specifically binding C5 and the VHH2 specifically binding C5 are anti-C5 VHHs binding different epitopes described herein. In some embodiments, the VHH specifically binding HTRA1 is an anti-HTRA1 VHH described herein.

In some embodiments, the bispecific antibody of the invention comprises or consists of a chain comprising from the N-terminus to the C-terminus:
the VHH1 specifically binding C5 described herein - the VHH2 specifically binding C5 described herein - the VHH3 specifically binding HTRA1 described herein,
the VHHs are linked by a linker.

In some embodiments, the VHH1 specifically binding C5 and the VHH2 specifically binding C5 are anti-C5 VHHs described herein which bind different epitopes.

In some embodiments, the VHH1 specifically binding C5
(i) comprises complementarity determining regions (CDRs) VHH CDR1, VHH CDR2 and VHH CDR3, wherein
   (a) VHH CDR1 comprises or consists of the amino acid sequence set forth in SEQ ID NO:4, VHH CDR2 comprises or consists of the amino acid sequence set forth in SEQ ID NOs:5 or 7 or 8, VHH CDR3 comprises or consists of the amino acid sequence set forth in SEQ ID NOs:6 or 30; or
   (b) VHH CDR1 comprises or consists of the amino acid sequence set forth in SEQ ID NO:4, VHH CDR2 comprises or consists of the amino acid sequence set forth in SEQ ID NO:5, VHH CDR3 comprises or consists of the amino acid sequence set forth in SEQ ID NO:6 or 30; or
   (c) VHH CDR1 comprises or consists of the amino acid sequence set forth in SEQ ID NO:4, VHH CDR2 comprises or consists of the amino acid sequence set forth in SEQ ID NOs: 7 or 8, VHH CDR3 comprises or consists of the amino acid sequence set forth in SEQ ID NO:6;
(ii) comprises a heavy chain variable region comprising or consisting of an amino acid sequence set forth in any one of SEQ ID NOs: 18-21, or an amino acid sequence having at least 90% identity thereto;
and VHH2 specifically binding C5
(i) comprises complementarity determining regions (CDRs) VHH CDR1, VHH CDR2 and VHH CDR3, wherein
   (a) VHH CDR1 comprises or consists of the amino acid sequence set forth in SEQ ID NO:1, VHH CDR2 comprises or consists of the amino acid sequence set forth in SEQ ID NO:2 or 9, VHH CDR3 comprises or consists of the amino acid sequence set forth in SEQ ID NO:3 or 10; or
   (b) VHH CDR1 comprises or consists of the amino acid sequence set forth in SEQ ID NO:1, VHH CDR2 comprises or consists of the amino acid sequence set forth in SEQ ID NO:2, VHH CDR3 comprises or consists of the amino acid sequence set forth in SEQ ID NO:3; or
   (c) VHH CDR1 comprises or consists of the amino acid sequence set forth in SEQ ID NO:1, VHH CDR2 comprises or consists of the amino acid sequence set forth in SEQ ID NO:9, and VHH CDR3 comprises or consists of the amino acid sequence set forth in SEQ ID NO:10.
(ii) comprises a heavy chain variable region comprising or consisting of an amino acid sequence set forth in any one of SEQ ID NOs: 17, 22 or 23, or an amino acid sequence having at least 90%, 91%, 92%, 93%, 94%, 95%, 96%, 97%, 98% or 99% identity thereto;
or vice versa.

In some embodiments, the VHH3 specifically binding HTRA1
(i) comprises complementarity determining regions (CDRs) VHH CDR1, VHH CDR2 and VHH CDR3, wherein
   (a) VHH CDR1 comprises or consists of the amino acid sequence set forth in SEQ ID NO:11, VHH CDR2 comprises or consists of the amino acid sequence set forth in SEQ ID NO:12, VHH CDR3 comprises or consists of the amino acid sequence set forth in SEQ ID NO:13; or
   (b) VHH CDR1 comprises or consists of the amino acid sequence set forth in SEQ ID NO:14, VHH CDR2 comprises or consists of the amino acid sequence set forth in SEQ ID NO:15, VHH CDR3 comprises or consists of the amino acid sequence set forth in SEQ ID NO:16; or
   (c) VHH CDR1 comprises or consists of the amino acid sequence set forth in SEQ ID NOs: 11 or 14, VHH CDR2 comprises or consists of the amino acid sequence set forth in SEQ ID NOs: 12 or 15, VHH CDR3 comprises or consists of the amino acid sequence set forth in SEQ ID NOs: 13 or 16;
(ii) comprises a heavy chain variable region comprising or consisting of an amino acid sequence set forth in SEQ ID NOs: 24 or 25, or an amino acid sequence having at least 90%, 91%, 92%, 93%, 94%, 95%, 96%, 97%, 98% or 99% identity thereto.

In some embodiments, the VHH1 specifically binding C5 comprises or consists of an amino acid sequence as set forth in SEQ ID NO:20.

In some embodiments, the VHH2 specifically binding C5 comprises or consists of an amino acid sequence as set forth in SEQ ID NO:23.

In some embodiments, the VHH3 specifically binding the HTRA1 comprises or consists of an amino acid sequence as set forth in SEQ ID NO:25.

In some particular embodiments, VHH1 comprises or consists of an amino acid sequence set forth in SEQ ID NO:20, VHH2 comprises or consists of an amino acid sequence set forth in SEQ ID NO:23, and VHH3 comprises or consists of an amino acid sequence set forth in SEQ ID NO:25.

In some embodiments, the linker comprises (G) ₙ, where n = 5 - 10, e.g., 5, 6, 7, 8, 9, 10. In some embodiments, the linker comprises or consists of the amino acid sequence of SEQ ID NO: 26.

In some embodiments, the bispecific antibody of the invention comprises a chain comprising or consisting of the amino acid sequence set forth in SEQ ID NO:27 or an amino acid sequence having at least 90%, 91%, 92%, 93%, 94%, 95%, 96%, 97%, 98% or 99% identity thereto.

In some embodiments, the bispecific antibody of the invention comprises or consists of the amino acid sequence set forth in SEQ ID NO:27 or an amino acid sequence having at least 90%, 91%, 92%, 93%, 94%, 95%, 96%, 97%, 98% or 99% identity thereto.

### VI. Polynucleotides, vectors and hosts

The invention provides nucleic acids encoding any one of the antibody molecules of the invention described above (anti-C5 antibodies or anti-HTRA1 antibodies or bispecific antibodies). A vector comprising the nucleic acid is also provided. In an embodiment, the vector is an expression vector (e.g., a pCDNA vector such as pCDNA3.1). Also provided is a host cell comprising the nucleic acid or the vector. In an embodiment, the host cell is eukaryotic. In another embodiment, the host cell is selected from the group consisting of a yeast cell, a mammalian cell (e.g. a CHO cell or a 293 cell, for example (HEK 293 or 293F cells or 293FT cells or Expi293 cells). In another embodiment, the host cell is prokaryotic.

In one aspect, the invention provides nucleic acids encoding any one of the above anti-C5 antibodies or anti-HTRA1 antibodies or bispecific antibodies.

To facilitate production and purification, an anti-C5 antibody or an anti-HTRA1 antibody or bispecific antibody may be fused at the N-terminus or the C-terminus (e.g., C-terminus) to a secretory signal peptide, and/or a tag peptide to facilitate purification, such as a hexahistidine tag or a biotin label.

As is clear to one skilled in the art, each antibody or polypeptide amino acid sequence may be encoded by a variety of nucleic acid sequences because of codon degeneracy.

In some embodiments, the nucleic acid of the present invention comprises a nucleic acid encoding amino acid sequence selected from any one set forth in SEQ ID NOs: 17-25 or 27 or 34, or a nucleic acid encoding an amino acid sequence having at least 85%, 90%, 91%, 92%, 93%, 94%, 95%, 96%, 97%, 98% or 99% identity to amino acid sequence selected from any one set forth in SEQ ID NOs: 17-25 or 27 or 34.

Nucleic acid sequences encoding molecules of the present invention can be generated using methods well known in the art, for example, by de novo solid phase DNA synthesis, or by PCR amplification.

### VII. Vectors and Host Cells

In an embodiment, one or more vectors comprising a nucleic acid of the present invention are provided. In an embodiment, the vector is an expression vector, such as a prokaryotic expression vector or a eukaryotic expression vector. Vectors include, but are not limited to, viruses, plasmids, cosmids, lambda phages, or yeast artificial chromosomes (YACs). In preferred embodiments, the expression vector is pCDNA, for example pCDNA3.1.

In an embodiment, a host cell comprising one or more polynucleotides of the invention is provided. In some embodiments, a host cell comprising an expression vector of the present invention is provided. Suitable host cells include prokaryotic microorganisms such as E. coli, eukaryotic microorganisms such as filamentous fungi or yeast, or various eukaryotic cells such as Chinese hamster ovary cells (CHO), insect cells, and the like. Suitable mammalian cell lines for suspension culture may be used. Examples of useful mammalian host cell lines include SV40 transformed monkey kidney CV1 line (COS-7); human embryonic kidney line (HEK 293 or 293F cells or 293FT cells or Expi293 cells), baby hamster kidney cells (BHK), monkey kidney cells (CV1), African green monkey kidney cells (VERO-76), human cervical cancer cells (HELA), canine kidney cells (MDCK), Buffalo rat liver cells (BRL 3A), human lung cells (W138), human liver cells (Hep G2), CHO cells, NSO cells, myeloma cell lines such as YO, NS0, P3X63, and Sp2/0, and the like. Mammalian host cell lines suitable for production of antibodies are known in the art. In a preferred embodiment, the host cell is a CHO or HEK293 cell or 293FT cell or Expi293 cell.

### VIII.Production and purification of molecules of the invention

In a further aspect, the invention provides a method for producing an antibody molecule (anti-C5 antibody or anti-HTRA1 antibody or bispecific antibody) of the invention comprising culturing a host cell comprising polynucleotides encoding the polypeptide chains under conditions suitable for expressing the polypeptide chains of the molecule, optionally further comprising assembling the polypeptide chain to produce the molecule under conditions suitable for assembling the polypeptide chain to the molecule.

For recombinant production, polynucleotides encoding polypeptide chains of the molecules of the invention may be inserted into one or more vectors for further cloning and/or expression in a host cell. Expression vectors can be constructed using methods well known to those skilled in the art. Expression vectors include, but are not limited to, viruses, plasmids, cosmids, lambda phages, or yeast artificial chromosomes (YACs). Once an expression vector for expression comprising one or more polynucleotides of the invention has been prepared, the expression vector can be transfected or introduced into a suitable host cell. A variety of techniques can be used to accomplish this purpose, for example, protoplast fusion, calcium phosphate precipitation, electroporation, transduction of retroviruses, viral transfection, gene gun, liposome-based transfection, or other conventional techniques.

The molecules prepared as described herein can be purified by known prior art techniques such as high performance liquid chromatography, ion exchange chromatography, gel electrophoresis, affinity chromatography (e.g., Protein A affinity chromatography), size exclusion chromatography, and the like. The actual conditions used to purify a particular protein will also depend on factors such as net charge, hydrophobicity, hydrophilicity, etc., and these will be apparent to one skilled in the art.

The purity of the molecules of the present invention can be determined by any one of a variety of well known analytical methods including size exclusion chromatography, gel electrophoresis, high performance liquid chromatography and the like. The physical/chemical properties and/or biological activities of the antibody molecules provided herein can be identified, screened or characterized by a variety of assays known in the art.

### IX. Assay

The molecules provided herein (anti-C5 antibodies or anti-HTRA1 antibodies or bispecific antibodies) can be identified, screened, or characterized for their physical/chemical properties and/or biological activities by a variety of assays known in the art.

The binding or dissociation properties of the anti-C5 antibodies or bispecific antibodies of the invention to human complement C5 protein may be determined by methods known in the art, such as bio-layer interferometry (BLI), ELISA, Western blot, ForteBio, and the like, or exemplary methods disclosed in Example 4 herein.

The hemolytic inhibiting effect of the anti-C5 or bispecific antibodies of the invention may be measured by methods known in the art, such as in vitro assays and/or cell assays. For example, a hemolysis experiment, such as the method set forth in Examples 5 or 6, can be used and the molecule is tested for hemolysis inhibition of the complement immune classical pathway and/or the complement immune alternative pathway.

For the determination of the biological activity of the anti-C5 antibody or the bispecific antibody of the present invention in blocking the effects of the complement classical or alternative pathway or blocking the production of C5a, methods known in the art can be used, such as detecting the content of C5a in the complement classical or alternative pathway-induced hemolysis system, such as the method shown in Example 7.

The therapeutic effect of the anti-C5 antibody or the bispecific antibody of the present invention can be determined by in vivo pharmacodynamic experiments of sodium dotate-induced geographic atrophy, such as the method shown in Example 16.

The effect of the anti-HTRA1 antibodies or bispecific antibodies of the invention in blocking HTRA1 enzymatic activity, for example serine protease activity or elastase activity, can be determined by an H2-Opt assay or an Elastase assay, such as the methods described in Examples 9 or 10.

### X. Immunoconjugates

In one aspect, the invention provides an immunoconjugate comprising the molecule of the invention (e.g., an anti-C5 antibody or an anti-HTRA1 antibody or bispecific antibody) and one or more other active ingredients (e.g., an active ingredient from a drug or therapeutic agent for treating a disease of the invention, such as a small molecule that enhances the therapeutic effect of the molecule of the invention).

### XI. Pharmaceutical Compositions

In one aspect, the invention provides a composition, e.g., a pharmaceutical composition, medicament or formulation, comprising the molecule of the invention (e.g., an anti-C5 antibody or an anti-HTRA1 antibody or bispecific antibody, or immunoconjugate, or the like).

In an embodiment, the composition further comprises pharmaceutical excipients, such as pharmaceutically-acceptable carriers, pharmaceutically-acceptable excipients, including buffers, as known in the art.

As used herein, a "pharmaceutically-acceptable carrier" includes any and all solvents, dispersion media, isotonic and absorption delaying agents, and the like that are physiologically compatible. For the use of pharmaceutical excipients and their uses, see also Handbook of Pharmaceutical Excipients, 8th Edition, R.C. Rowe, P.J. Seskey, and S.C. Owen, Pharmaceutical Press, London, Chicago.

The compositions or medicaments or formulations of the present invention can be in a variety of forms. These include, for example, liquid, semi-solid, and solid dosage forms such as liquid solutions (e.g., injections), powders or suspensions, liposomes, and suppositories. Preferred forms depend on the intended mode of administration and therapeutic use.

The compositions or drugs or formulations of the present invention may also include, in addition to one or more molecules of the present invention, other therapeutic agents that are required for the particular indication being treated, preferably that do not adversely affect the activity of each other. Thus, in an embodiment, a composition or formulation or medicament, e.g., a pharmaceutical composition, comprises one or more molecules of the present invention, in combination with one or more other therapeutic agents.

The compositions or medicaments or formulations of the present invention can be in a variety of forms. These include, for example, liquid, semi-solid, and solid dosage forms such as liquid solutions (e.g., injections), powders or suspensions, liposomes, and suppositories. The compositions or drugs or formulations of the present invention are suitable for intravenous, intramuscular, subcutaneous, parenteral, rectal, spinal cord, or dermal administration (e.g., by injection or infusion). Preferred forms depend on the intended mode of administration and therapeutic use.

### XII. Pharmaceutical combinations and Kits

The invention also provides a pharmaceutical combination or pharmaceutical combination product comprising the molecule of the invention (anti-C5 antibody or anti-HTRA1 antibody or bispecific antibody or immunoconjugate thereof). Optionally, the pharmaceutical combination or pharmaceutical combination product further comprises one or more other therapeutic agents.

The invention also provides a kit comprising the pharmaceutical combination, for example within the same package:
- a first container containing a pharmaceutical composition comprising the molecule of the invention (anti-C5 antibody or anti-HTRA1 antibody or bispecific antibody or immunoconjugate thereof);
- Optionally, a second container containing the pharmaceutical composition comprising one or more other therapeutic agents (in some embodiments, two or more other therapeutic agents are in the same container, or in different containers, respectively).

### XIII. Uses and Methods

An aspect of the invention provides a method of preventing or treating a complement system and/or HTRA1-related disease or condition in a subject, comprising administering to the subject an effective amount of the molecule of the invention (e.g., an anti-C5 antibody or an anti-HTRA1 antibody or bispecific antibody or immunoconjugate thereof, and the like), or a composition or medicament or formulation comprising thereof.

In some embodiments, the present invention provides a method of preventing or treating a complement system-related disease or condition in a subject, comprising administering to the subject an effective amount of a molecule of the present invention (e.g., an anti-C5 antibody or bispecific antibody or immunoconjugate thereof, and the like), or a composition or medicament or formulation comprising same.

In some embodiments, the present invention provides a method of preventing or treating a HTRA1-related disease or condition in a subject, comprising administering to the subject an effective amount of a molecule of the present invention (e.g., an anti-HTRA1 antibody or bispecific antibody or immunoconjugate thereof, and the like), or a composition or medicament or formulation comprising same.

An aspect of the invention provides a method of preventing or treating a complement system-related and HTRA1-related disease or condition in a subject, comprising administering to the subject an effective amount of the molecule of the invention (e.g., an anti-C5 antibody or an anti-HTRA1 antibody or bispecific antibody or immunoconjugate thereof, and the like), or a composition or medicament or formulation comprising thereof.

In some embodiments, the complement system-related disease is caused by abnormal activation of the complement system or dysregulation of the complement system. In some embodiments, the disease treatment will benefit from inhibiting the activity of the complement system.

In some embodiments, the complement system-related disease or condition is a complement C5-related disease or condition. In some embodiments, the subject has complement C5 or C5a protein (e.g., at an elevated levels, e.g., at nucleic acid or protein levels) (e.g., as compared to a healthy subject). In some embodiments, the subject has complement C5 or C5a (e.g., at an elevated level, e.g., at nucleic acid or protein level) in a biological sample (e.g., as compared to a biological sample of a healthy subject). In some embodiments, the complement system-related disease treatment would benefit from inhibiting complement C5 or C5a at the nucleic acid or protein level, or from blocking production of C5a.

In some embodiments, the complement-related disease or condition may be a disease that requires inhibition of hemolysis, for example, a disease that requires inhibition of hemolysis of the complement immune classical pathway and/or the complement immune alternative pathway.

In some embodiments, in the HTRA1-related disease or condition, the subject has HTRA1 (e.g. at an elevated level, e.g. at a nucleic acid or protein level) (e.g. as compared to a healthy subject). In some embodiments, the subject has HTRA1 (e.g. at an elevated level, e.g. at a nucleic acid or protein level) in a biological sample (e.g. as compared to a biological sample of a healthy subject). In some embodiments, the treatment of the HTRA1-related disease or condition will benefit from HTRA1 inhibition at the nucleic acid or protein level.

In some embodiments, the biological sample is a body fluid, such as vitreous humor or blood.

In some embodiments, the HTRA1-related disease or condition is a disease that requires inhibition of the activity of HTRA1, for example its serine protease activity and/or elastase activity.

In some embodiments, the disease or condition is an ocular disease or condition.

In some embodiments, the molecules or compositions or drugs or formulations or combination products of the invention comprising thereof delay the onset of the condition and/or symptoms associated with the condition.

In some embodiments, the molecules or compositions or medicaments or formulations of the invention comprising thereof can also be administered in combination with one or more other therapies, e.g., therapeutic modalities and/or other therapeutic agents, for use as described herein, for example, for the prevention and/or treatment of the related diseases or conditions referred to herein.

The routes and methods of administration of the molecules or compositions or drugs or formulations or combination products comprising thereof of the present invention are according to known methods, for example, injection or infusion, for example by intravitreal injection, for example by intravitreal injection.

In other aspects, the present invention provides the use of a molecule of the present invention or a composition or combination product comprising the thereof, in the manufacture or preparation of a medicament for use as described herein, for example, in the prevention or treatment of a related disease or condition as referred to herein.

In other aspects, the invention also provides the molecule of the invention (e.g., an anti-C5 antibody or an anti-HTRA1 antibody or bispecific antibody or immunoconjugate thereof, and the like), or a composition or drug or formulation or combination product comprising thereof, for use in therapy.

In other aspects, the invention also provides the molecule of the invention (e.g., an anti-C5 antibody or an anti-HTRA1 antibody or bispecific antibody or immunoconjugate thereof, and the like), or a composition or drug or formulation or combination product comprising thereof, for use in the treatment of a related disease or condition referred to herein.

### XIIII. Diagnosis And Detection

In certain embodiments, the molecules provided herein (e.g., an anti-C5 antibody or an anti-HTRA1 antibody or bispecific antibody or immunoconjugate thereof and the like) can be used for detecting the presence of complement C5 and/or HTRA1 in a biological sample.

The term "detection" as used herein, including quantitative or qualitative detection, exemplary detection methods may involve immunohistochemistry, immunocytochemistry, flow cytometry (e.g., FACS), magnetic beads in complex with antibody molecules, ELISA assays, PCR-techniques (e.g., RT-PCR). In certain embodiments, the biological sample is a body fluid.

In certain embodiments, the methods comprise contacting a biological sample with a molecule as described herein, for example an anti-C5 antibody or bispecific antibody or immunoconjugate thereof, under conditions which allow it to bind to complement C5 and detecting whether a complex is formed between the molecule and complement C5, wherein the formation of the complex is indicative of the presence of complement C5. The method can be an in vitro or in vivo method. In an embodiment, the molecules of the invention are used to select a subject suitable for treatment with an inhibitor against complement C5 (e.g., an antibody against complement C5, such as the anti-C5 antibody or the bispecific antibody of the invention or an immunoconjugate thereof, etc.), for example where complement C5 is a biomarker for selection of the subject.

In certain embodiments, the methods comprise contacting a biological sample with a molecule as described herein (e.g. an anti-HTRA1 antibody or bispecific antibody or immunoconjugate thereof) under conditions which allow it to bind to HTRA1 and detecting whether a complex is formed between the molecule and HTRA1, wherein the formation of the complex represents the presence of complement HTRA1. The method can be an in vitro or in vivo method. In an embodiment, the molecules of the invention are used to select subjects suitable for treatment with an inhibitor against HTRA1 (e.g., an antibody against HTRA1, for example the anti-HTRA1 antibody or the bispecific antibody of the invention or an immunoconjugate thereof, and the like), for example where HTRA1 is a biomarker for the selection of said subjects.

In certain embodiments, the methods comprise contacting a biological sample with a molecule as described herein, for example a bispecific antibody or an immunoconjugate thereof, under conditions which allow it to bind to complement C5 and/or HTRA1 and detecting whether a complex is formed between the molecule and complement C5 or HTRA1, wherein the formation of the complex is indicative of the presence of complement C5 and/or HTRA1. The method can be an in vitro or in vivo method. In an embodiment, the molecules of the invention are used to select a subject suitable for treatment with an inhibitor against complement C5 and/or HTRA1, (e.g., an antibody against complement C5 and/or HTRA1, for example the anti-C5 antibody or the anti-HTRA1 antibody or the bispecific antibody of the invention or an immunoconjugate thereof, and the like), for example wherein complement C5 and/or HTRA1 is a biomarker for selection of said subject.

In some embodiments, the biological sample is a body fluid, such as vitreous humor or blood.

In certain embodiments, labeled molecules of the invention are provided (e.g. anti-C5 antibodies or anti-HTRA1 antibodies or bispecific antibodies or immunoconjugates thereof and the like of the invention). The label includes, but is not limited to, labels or moieties that are detected directly, such as fluorescent labels, chromophore labels, electron-dense labels, chemiluminescent labels, and radioactive labels, and moieties that are detected indirectly, such as enzymes or ligands, for example, by enzymatic reactions or molecular interactions.

In some embodiments, the marker is for example a biotin or His tag.

In some embodiments provided herein, the sample is obtained prior to treatment with the molecule of the invention or a composition, medicament, formulation or combination product comprising thereof. In some embodiments, the sample is obtained prior to use of other therapies. In some embodiments, the sample is obtained during or after treatment with other therapies.

In some embodiments, complement C5 and/or HTRA1 is detected prior to treatment, for example, prior to initiation of treatment or prior to a treatment following a treatment interval.

In some embodiments, provided is a method of treating a disease of the present invention, comprising examining a subject (e.g., a sample) (e.g., a subject sample) for the presence of complement C5 and/or HTRA1, thereby determining a complement C5 and/or HTRA1 value, comparing the complement C5 and/or HTRA1 value to a reference value (e.g., a value in a normal individual), and if the complement C5 and/or HTRA1 value is greater than the reference value, administering to the subject a therapeutically effective amount of the molecule of the present invention, or composition, medicament or formulation comprising thereof, optionally in combination with one or more other therapies, thereby treating the disease.

Any or all of the features described above and throughout this application may be combined in various embodiments of the invention. The following examples further illustrate the invention, however, it is to be understood that the examples are for the purpose of illustration and should not be construed as constituting any limitation.

### Examples

### Example 1. Preparation and panning of C5 phage display library

1.1 Two healthy adult alpacas (Chengdu Apak Company) were selected, and 0.5 or 0.25 mg of recombinant protein antigen C5 (Beijing ACRO) was mixed with Freund's complete or incomplete adjuvant at a ratio of 1:1. Alpacas were immunized by subcutaneous multi-point injection on the back for a total of five times at an interval of 2-3 weeks.

1.2 50 mL of peripheral blood was collected from alpacas, and lymphocytes were isolated. 1 mL of Trizol reagent (Thermo) was added per 2.5 × 10 ⁷ viable cells, and total RNA was extracted by chloroform/isopropanol precipitation method. 10 µg of RNA was taken as a template for reverse transcription using PrimeScript reverse transcription kit (Takara). The cDNA was used as a template for the first round of PCR reaction, and the forward primer Alp-VhL and the reverse primer Alp-2b/2cR were used to obtain the first round of PCR products. The second-round PCR reaction was performed using the first-round PCR product as a template, and the forward primer Alp-VhF and the reverse primer Alp-JHR-SalI were used to obtain the second-round PCR product. The pC3-HF vector and the second-round PCR products were double digested with SacI and SalI (Thermo), respectively, and the digested products were added to T4 ligase (Thermo) for ligation. The ligation products were electrotransformed into TG1 competent cells to construct the VHH antibody immune library.

The above library bacterial solution was inoculated into 100 mL of YT-AG medium (Sangon Biotech Co., Ltd.) and cultured until the logarithmic growth phase. M13KO7 helper phage was added for infection, and the bacteria were resuspended with 2× YT-AK medium (Sangon Biotech Co., Ltd.) and cultured at 30 °C and 200 rpm overnight. Culture supernatant was collected and recombinant phages were prepared by PEG/NaCl precipitation.

1.3 Recombinant phages were subjected to 2-3 rounds of panning experiments using biotin-labeled antigen C5 (C5 from ACRO, biotin-labeled in-house). 30 µL of Pierce streptavidin Magnetic Beads (Thermo) and 100 pMoL of biotin-labeled antigen were added to each tube and incubated at room temperature for 30 min. Then 1 × 10 ¹² cfu of recombinant phage was added and incubated at room temperature for 1 h. The obtained mixture was washed 10-15 times with 1 mL of 0.1%-0.05% PBST, and finally, 0.5 mL of pH 2.5 glycine buffer was added to elute antigen-binding recombinant phages, which were then seeded on YT-AG plates to infect the TG1 in the logarithmic growth phase, and incubated overnight. At the same time, a small amount of the infected bacterial solution was gradiently diluted and spread on a YT-AG plate to form monoclones, which were used for the binding ELISA test in Section 1.4. The next day, the bacterial lawn was scraped off the plate and recombinant phages were prepared as described in Section 1.2 for the next round of panning experiments.

1.4 Binding ELISA test for binding activity and clone sequencing.

The monoclotnal plates in Section 1.3 were taken, the monoclone was picked and inoculated into a 96-deep-well plate containing YT-A. When the OD600 was about 0.5, IPTG at a final concentration of 1 mM was added to induce expression overnight at 30 °C. Polymyxin was added to the culture supernatant and the mixture was incubated at 37 °C for 30 min. After centrifugation, the supernatant was used for binding ELISA test.

C5 antigen (Beijing ACRO) was diluted with PBS buffer to 0.5 µg/mL to coat a 96-well ELISA plate. The antigen-coated plate was washed 3 times with PBST, added with the blocking reagent to 300 µL/well, and allowed to stand at room temperature for 1 h for blocking. The plate was washed 3 times with PBST, added with 80 µL of blocking reagent + 20 µL of bacterial expression supernatant, and incubate at room temperature for 1 h.

The plate was washed 3 times with PBST, added with 100 µL/well of Anti-Flag/HRP secondary antibody (Sigma) diluted with blocking reagent, and incubated at room temperature for 1 h. The plate was washed 6 times with PBST, added with TMB developing solution to 100 µL/well, and developed color away from light for 5-15 min. Then 100 µL/well of the stop solution was added. The absorbance at OD450 nm was measured using a microplate reader, and bacterial clones with a reading greater than 0.5 were selected and sent to GENEWIZ for sequencing.

### Example 2. Panning of the HTRA1 phage display library

2.1 The HTRA1 recombinant antigen (UniProtKB/Swiss-Prot: Q92743) customized by Sino, Beijing, based on our company's VHH synthesis library was used for 2-3 rounds of solid phase panning. Specifically, 30 pmoL of HTRA1 recombinant antigen was added to each ELISA well and coated overnight at 4 °C. The plate was washed 3 times with PBST and blocked with the blocking buffer at room temperature for 1 h. Each ELISA well was added with 5x10 ¹¹ cfu of recombinant phage, and incubated at room temperature for 1 h. After incubation, the phages were washed 10-15 times with 0.1%-0.05% PBST for 3-5 min each time. Finally, antigen-binding recombinant phages were eluted with pH 2.5 glycine buffer, used to infect the TG1 at logarithmic-phase, and the TG1 was plated on YT-AG plates for culture overnight. At the same time, a small amount of the inoculated bacterial solution was taken, subjected to gradient dilution, and plated on a YT-AG plate to form monoclones on the plate, which were used for the binding ELISA test in Section 2.2. The next day, the bacterial lawn was scraped off the plate and recombinant phages were prepared as described in Section 1.2 for the next round of panning experiments.

2.2 Binding ELISA test for binding activity and clone sequencing.

The monoclonal plates in Section 2.1 were taken, the monoclone was picked and inoculated into a 96-deep-well plate containing YT-A. When the OD600 was about 0.5, IPTG at a final concentration of 1 mM was added to induce expression overnight at 30 °C. Polymyxin was added to the culture supernatant and the mixture was incubated at 37 °C for 30 min. After centrifugation, the supernatant was used for binding ELISA test.

HTRA1 antigen (customized by Sino, Beijing) was diluted with PBS buffer to 0.5 µg/mL to coat a 96-well ELISA plate, and the plate was placed in a refrigerator at 4 °C overnight. The antigen-coated plate was washed 3 times with PBST, added with the blocking reagent to 300 µL/well, and allowed to stand at room temperature for 1 h for blocking. The plates were washed 3 times with PBST, added with 80 µL of blocking reagent + 20 µL of the above TG1 monoclonal expression supernatant, and incubated at room temperature for 1 h.

The plate was washed 3 times with PBST, added with 100 µL/well of Anti-Flag/HRP secondary antibody (Sigma) diluted with blocking reagent, and incubated at room temperature for 40 min. The plate was washed 6 times with PBST, added with TMB developing solution to 100 µL/well, and developed color away from light for 5-15 min. Then add 100 µL/well of the stop solution was added. The absorbance at OD450 nm was measured using a microplate reader, and bacterial clones with a reading greater than 0.5 were selected and submitted to GENEWIZ for sequencing.

### Example 3. Eukaryotic expression and purification of candidate antibodies

In the present invention, the VHH antibody sequences in the anti-C5 or HTRA1 positive phage obtained from Example 1 were amplified by PCR and inserted into His-tag (HHHHHH) pCDNA3.1 using molecular biology techniques, transfected into HEK-293 (Thermo Fisher Scientific), expressed and purified to finally obtain the VHH-His recombinant antibody with a 6XHis tag at the C-terminus. The VHH antibodies individually validated mentioned below are all VHH-His recombinant antibodies.

The purified antibodies were screened by complement hemolysis assay (CH50, ACH50) or enzyme activity assay (H2-Opt assay, Elastase assay), and finally 2 anti-C5 VHH antibodies (1,293) and 1 anti-HTRA1 antibody SA239 were obtained.

The full-length amino acid sequences of the anti-C5 VHH antibody (1,293) and one anti-HTRA1 antibody (SA239) obtained in the present invention, as well as the specific sequences, are shown in the sequence list.

### Example 4 Determination of Binding Kinetics of VHH Antibodies of the Present Invention to Antigen by Bio-layer Interferometry

Equilibrium dissociation constants (KD) of the antibodies of the invention binding to human C5 or HTRA1 were determined using bio-layer interferometry (BLI). Experimental assays were performed using ForteBio Octet Red96e instrument and affinity measurements were carried out according to current methods (Estep, P et al., High throughput solution Based measurement of antibody-antigen affinity and epitope binning. MAbs, 2013.5(2): pp. 270-8).

Half an hour before the start of the experiment, an appropriate number of SA sensors (18-5019, Satorius) were soaked in SD buffer (PBS 1×, BSA 0.1%, Tween-20 0.05%) according to the number of samples.

100 µL of SD buffer, antibody, and biotin-labeled antigens (including human C5 and human HTRA1) were added to a 96-well half-size polystyrene black microplate (Greiner, 675076), respectively. The plate according to the sample position was arraged, and the sensor position was selected. The instrument parameters were set as follows: running steps: Baseline, Loading ~1 nm, Baseline, Association, and Dissociation; the running time of each step depended on the binding and dissociation rates of the sample, with a rotation speed of 400 rpm and a temperature of 30 °C. K _{D} values were analyzed using ForteBio analysis software.

In the experiment described in the above assay, the affinity of the antibody is shown in Table 1:

**Table 1. Affinity constant (equilibrium dissociation constant) of monovalent binding of C5 antigen-antibody detected by ForteBio**

| Clone ID | Human Complement C5 | | | Cyno Complement C5 | | | Human C5(R885H) | | Complement |
|---|---|---|---|---|---|---|---|---|---|
| | KD (M) | Kon (1/Ms) | kdis(1/s ) | KD (M) | Kon (1/Ms) | kdis(1/s ) | KD (M) | Kon (1/Ms) | kdis(1/s ) |
| 1 | 1.75E-1 0 | 1.37E+0 6 | 2.39E-0 4 | 2.19E-1 0 | 3.18E+0 6 | 6.97E-0 4 | 1.33E-1 0 | 1.69E+0 6 | 2.24E-0 4 |
| 293 | 7.23E-1 0 | 1.75E+0 6 | 1.27E-0 3 | 1.35E-0 9 | 4.62E+0 6 | 6.26E-0 3 | 1.83E-0 9 | 2.32E+0 6 | 4.24E-0 3 |

**Table 2. Affinity constant (equilibrium dissociation constant) of bivalent binding of HTRA1 antigen-antibody detected by ForteBio**

| Clone ID | KD (M) | Kon (1/Ms) | Kdis (1/s) |
|---|---|---|---|
| SA239 | 8.608E-10 | 1.164E+6 | 1.002E-3 |

In the above tests, the monovalent KD values of antibodies 1,293 to human C5 were 1.75E-10 M and 7.23E-10 M, respectively, the monovalent affinity KD values to cyno C5 were 2.19E-10 M and 1.35E-09 M, respectively, and the monovalent affinity KD values to human C5 (R885H) were also 1.33E-10 M and 1.83E-09 M, respectively.

The KD of the bivalent affinity of antibody SA239 to human HTRA1 was 8.608E-10 M.

### Example 5. Hemolysis test induced by anti-C5 VHH antibodies blocking the complement classical pathway (CH50)

The use of sensitized sheep red blood cells (immune complexes) can activate the complement classical pathway, leading to the formation of transmembrane pores on the surface of red blood cells, allowing extracellular water to penetrate, causing red blood cells to swell and cause hemolysis. In this study, a series of concentrations of anti-C5 VHH antibodies were fully reacted with a certain amount of complement, and then reacted with sensitized sheep red blood cells to verify the blocking effect of anti-C5 VHH antibodies on the complement classical pathway.

0.825 g of anhydrous calcium chloride, 5 g of magnesium chloride hexahydrate were weighed and dissolved in 50 mL of GVB buffer to obtain a 1000X calcium and magnesium ion solution, which was stored at 4 °C. 500 mL of GVB buffer was added with 500 µL of 1000X calcium and magnesium ion solution to obtain Gelatin Veronal Buffer (GVB). A vial of human serum complement dry powder (Sigma) was dissolved in 1 mL of ultrapure water, and then diluted with 9 mL of Gelatin Veronal Buffer, which was stored at 4 °C for later use. 10 mL of sheep erythrocytes (Kejing Biological Technology Co., Ltd.) was pipepted into a 50 mL centrifuge tube and 30 mL of 1X PBS was added. The mixture was centrifuged at 500 g for 10 min at 4 °C, and the supernatant was discarded. The washing was repeated until the supernatant was clear. The centrifuged red blood cells were added with 5 mL of 1X PBS, gently pipetted up and down to mix well, and diluted 10,000 times. Then, 10 µL of the diluted red blood cells were mixed well with 10 µL of Trypan Blue Stain (0.4%), and counted with a cell counting plate. The red blood cells were diluted to 1 × 10E9 cells/mL with 1 × PBS, and the hemolysin was diluted 1000-fold with 1 × PBS. The two solutions were mixed well in equal volume and placed on a CO₂ shaker at 37 °C, 120 r/min, for 45 min. Then, the plate was placed in an ice bath for 45 min. Red blood cells were centrifuged at 500 g and 4 °C for 10 min, the supernatant was discarded, and 1×PBS was added to 40 mL. The cells were centrifuged at 500 g and 4 °C for 10 min, the supernatant was discarded, and the washing was repeated twice. The sensitized sheep erythrocytes were finally resuspended in 5 mL of 1X PBS. The sensitized red blood cells were diluted 10,000-fold, and 10 µL of the diluted red blood cells were mixed with 10 µL of Trypan Blue Stain (0.4%), followed by counting with a cell counting plate. The red blood cells were diluted to 1 × 10E8 cells/mL with Gelatin Veronal Buffer and placed on ice for later use. The antibodies to be tested (test samples: Control: IgG1 antibody; ARC1905 (Beixin Biotechnology); VHH antibody 1 and VHH antibody 293) were serially diluted 3-fold with Gelatin Veronal Buffer. The starting concentration was 200 nM. Human complement serum was diluted 5-fold with Gelatin Veronal Buffer.

100 µL of sample and 50 µL of diluted human complement serum were added to each well.

For the positive control group (data not shown), 100 µL of Gelatin Veronal Buffer and 50 µL of diluted human complement serum were added to each well.

For the negative control group (data not shown), each well was the same as that in the positive control group, and no subsequent contact reaction with sheep red blood cells was observed. 37 °C shaker, 120 r/min, 45 min.

Sensitized sheep red blood cells at a concentration of 1×10E8 cells/mL were added at 50 µL per well. 37 °C shaker, 120 r/min, 30 min. The mixture was pippetted up and down and then put back, 37 °C shaker, 120 r/min, 30 min. The mixture was pipetted up and down, and centrifuged at 1000 g and 15 °C for 3 min. 100 µL of the supernatant was pipetted into a 96-well plate and the OD405 nm value was read by a microplate reader.

The calculated Inhibition of hemolysis by erythrocyte was as follows: 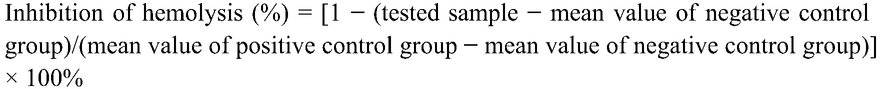 Inhibition of hemolysis (%) = [1 - (tested sample - mean value of negative control group)/(mean value of positive control group - mean value of negative control group)] × 100%

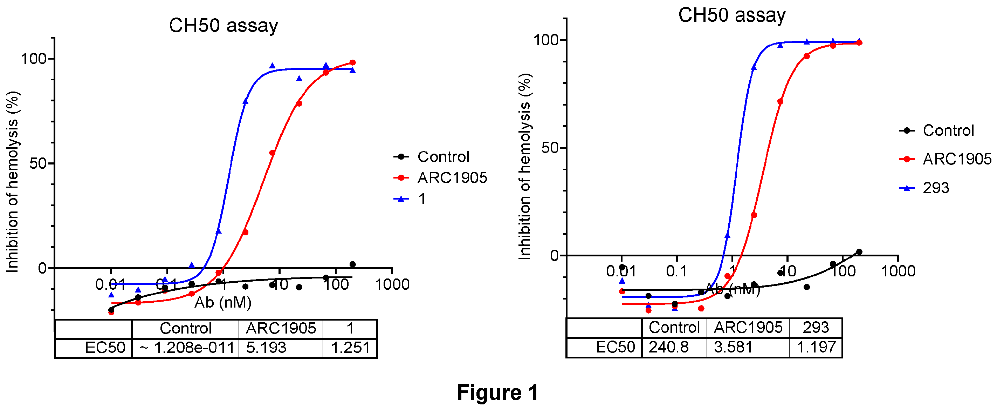

The blocking results of the two anti-C5 VHH antibodies obtained in the present invention are shown in Fig. 1, and the antibodies of candidate molecules 1 and 29 can block the complement classical pathway.

### Example 6. Hemolysis test induced by anti-C5 VHH antibody blocking the complement alternative pathway (ACH50)

When the complement alternative pathway (AP) is activated, C1, C2, and C4 are not involved, while C3, C5-C9, and factors B, D, P, H, and I are involved. Chelating Ca2+ in samples with EGTA can block C1 and thus block the traditional complement pathway activation. The rabbit red blood cells that can activate factor B are added to activate the alternative complement pathway, i.e., leading to hemolysis of rabbit red blood cells. In this study, based on the above principles, the tested samples at different concentration gradients were reacted with complements and MgEGTA, and then rabbit red blood cells were added to observe the hemolysis reaction, so as to validate the blocking effect of anti-C5 VHH antibodies on the alternative complement pathway.

5 mL of rabbit peripheral blood (Suzhou Huqiao Biotechnology Co., Ltd ) was transferred into a 50 mL centrifuge tube, added with 35 mL of PBS, and centrifuged at 400 g and 4 °C for 10 min. The supernatant was discarded, and washing was repeated until the supernatant was clear. Rabbit red blood cells were resuspended in 5 mL of PBS and diluted 2000-fold. Then, 10 µL of the diluted rabbit red blood cells were mixed well with 10 µL of Trypan Blue Stain (0.4%), and counted under an inverted microscope using a hemocytometer. Rabbit red blood cells were placed on ice for later use. 2 vials of human serum complement dry powder was taken, added with 4 mL of GVB buffer, and gently shaked to completely dissolve. Each tested sample (tested samples: Control: IgG1 antibody; ARC1905; VHH antibody 1 and VHH antibody 293) was serially diluted 3-fold with GVB buffer, with an initial concentration of 6000 nM.

According to the arrangement order of 96-well plates, 25 µL of human serum complement, 75 µL of sample, 40 µL of GVB buffer, and 10 µL of 0.1 mol/L MgEGTA were added to each well sequentially.

Positive control group (data not shown): 25 µL complement, 115 µL GVB, 10 µL 0.1 mol/L MgEGTA per well;
Negative control group (data not shown): same as the positive control group, but no subsequent contact reaction with rabbit red blood cells. 37 °C thermostatic shaker, 120 r/min, 45 min. Rabbit red blood cells were diluted with GVB buffer to obtain a suspension with a concentration of 1 × 10E8 cells/mL, and 50 µL of the suspension was added to each well. In addition, 200 µL of rabbit red blood cells were pipetted into 3 empty wells.

37 °C thermostatic shaker, 120 r/min, 30 min, the mixture was taken out and mixed well by pipetting up and down, and put back in 37 °C thermostatic shaker, 120 r/min, 30 min. The tube was taken out, mixed well by pipetting up and down, and centrifuged at 1000 g and 15 °C for 3 min. 50 µL of the supernatant containing only rabbit erythrocytes was pipetted into the negative control group and mixed well. 100 µL of the supernatant was pipetted into a new 96-well plate and the OD405 nm value was read by a microplate reader.

The calculated Inhibition of hemolysis by the erythrocyte were as follows: 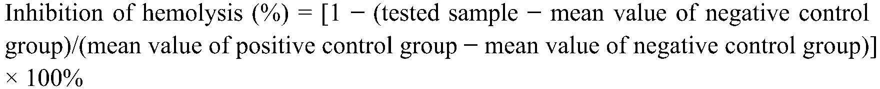 Inhibition of hemolysis (%) = [1 - (tested sample - mean value of negative control group)/(mean value of positive control group - mean value of negative control group)] × 100%

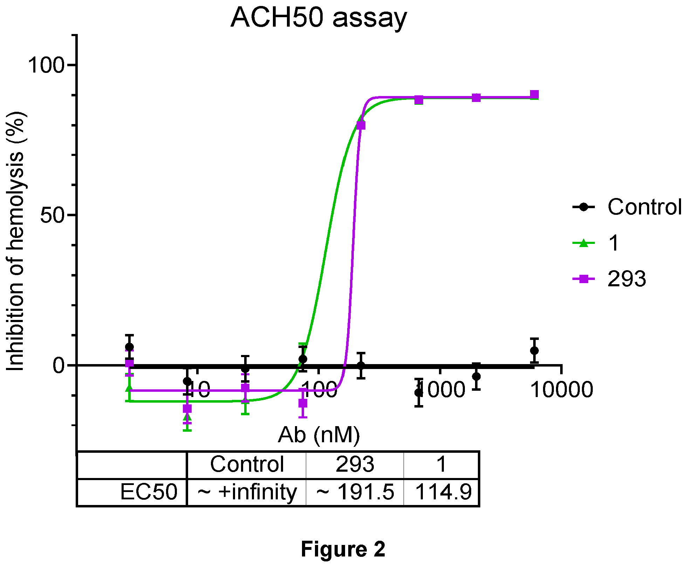

The blocking results of the two anti-C5 VHH antibodies obtained in the present invention are shown in Fig. 2, and the antibodies of candidate molecules 1 and 293 can block the complement alternative pathway.

### Example 7 Anti-C5 VHH Antibodies Blocking Complement Classical Pathway C5a Production (C5a ELISA)

In the classical and alternative complement pathways, C5 interacts with C5 convertase to form C5a and C5b, which go down to form the final MAC complex to lyse cells, while C5a can interact with C5a receptors to participate in biological activities. In this study, the Human C5a ELISA Kit II (BD, cat. no.: 557965) was used to determine the C5a content in the tested samples in the complement classical or alternative pathway-induced hemolysis system, so as to validate the biological activity of anti-C5 VHH antibody in blocking C5a production.

The kit was taken out from the 4 °C refrigerator and placed at room temperature for half an hour. The sample to be tested (supernatant obtained in Example 5: CH50 supernatant) was taken out, and the CH50 supernatant was diluted 2-fold with Standard/Sample Diluent. 50 µL of ELISA Diluent was added to the antibody-coated plate. The sample was added at 100 µL/well, sealed with a sealing film, and incubated at room temperature for 2 h. 48 µL Enzyme Concentrate was diluted with 12 mL Detection Antibody. The plate was washed 3 times with 300 µL of Wash Buffer per well, and the residual liquid was removed by pressing the plate on a paper towel for the last time. 100 µL of Working Detector was added to each well, sealed with a sealing film, and incubated at room temperature for 1 h. Wash buffer was added at 300 µL/well and the plate was washed 3 times, and the residual liquid was removed by pressing the plate on a paper towel for the last time. 100 µL of TMB One-Step Substrate Reagent was added to each well and incubated for 30 min at room temperature in the dark. 50 µL of Stop Solution was added to each well, and gently tapped to mix well in dark. The fluorescence reading was performed using a multifunctional microplate reader, with settings: Optical Configuration as Monochromator, Read Mode as ABS, Read Type as Endpoint, 2 wavelengths (450 nm and 620 nm), Plate Type as 96 Well Standard clrbtm, Shake as Off, and Read Order as Row.

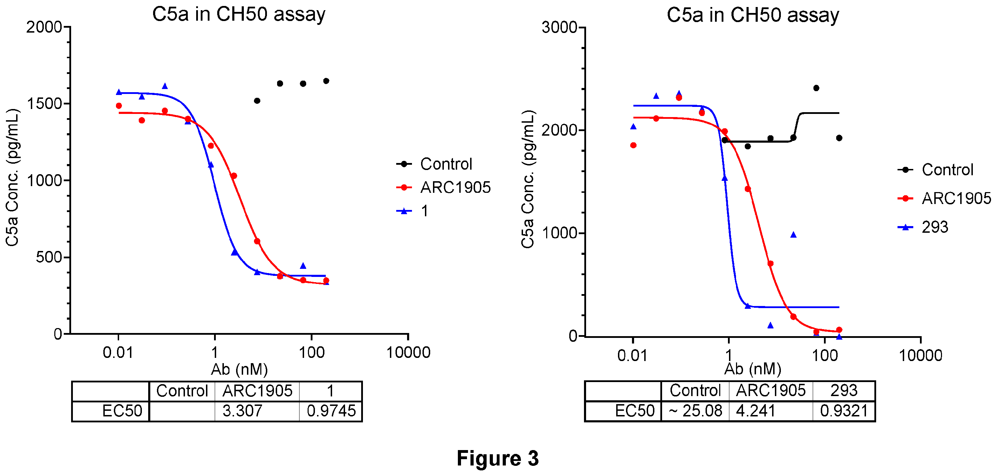

The blocking results of the two anti-C5 VHH antibodies obtained in the present invention are shown in Fig. 3. The antibodies of candidate molecule 1, 293 can block the production of C5a in the complement classical pathway.

### Example 8 Anti-C5 VHH antibody binding epitope analysis

The antibodies of the present invention bound to human C5 (ACRO, Beijing) was determined for epitope difference by bio-layer interferometry (BLI) and grouped. The ForteBio Octet Red96e instrument was used for the test. Half an hour before the start of the test, an appropriate number of SA sensors (18-5019, Satorius) were soaked in SD buffer (PBS 1×, BSA 0.1%, Tween-20 0.05%) according to the number of samples.

The samples to be tested were divided into first Ab (for saturated antigen) and second Ab (for competitive binding). 100 µL of SD buffer, antibody, and biotin-labeled antigens (including human C5) were added to a 96-well half-size polystyrene black microplate (Greiner, 675076), respectively. The plate was arragened according to the sample position, and the sensor position was selected. The instrument parameters were set as follows: running steps: Baseline, loading antigen at about 1 nm, Baseline, loading first Ab to saturation, second Ab Association; the running time of each step depended on the binding and dissociation rates of the samples, with a rotation speed of 1000 rpm and a temperature of 30 °C. The second Ab that competes with the first Ab for binding binds to similar antigen epitopes. The Second Ab does not compete or partially compete with the First Ab, and the antigen-binding epitope is different.

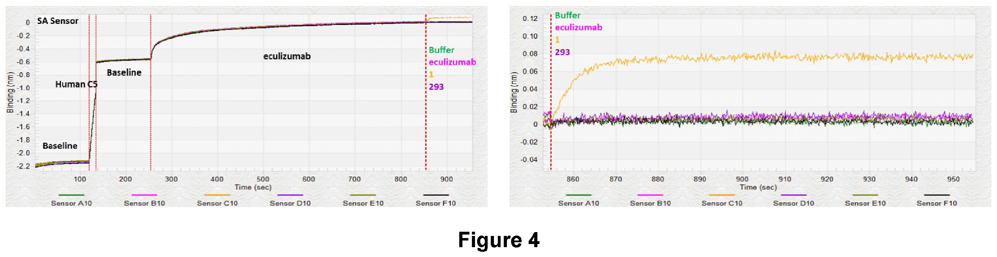

The binding epitope results of the 2 anti-C5 VHH antibodies obtained in the present invention are shown in Figure 4. The epitopes of candidate 1,293 antibodies are different.

### Example 9. H2-Opt assay by anti-HTRA1 VHH antibody blocking

HTRA1 is a serine protease. The polypeptide Mca-IRRVSYSF(K-Dnp)K (abbreviated as H2-Opt) can be cleaved by HTRA1 to produce fluorescent products. The generation rate of these products can be detected with a microplate reader to indicate the amount of active HTRA1 in the system, and this assay was used to verify the blocking activity of the anti-HTRA1 VHH antibody against HTRA1-mediated cleavage of H2-Opt.

Reaction buffer B was prepared by dissolving 5.85 g of sodium chloride, 1.25 g of CHAPS, and 3.0 g of Tris base in 400 mL of ultrapure water. The pH was adjusted to 8.0, and the solution was brought to a final volume of 500 mL. The resulting buffer was filtered through a 0.22 µm filter and stored at 4°C in refrigerator. The H2-Opt stock solution (10 mM) was prepared by dissolving 5 mg of H2-optimal substrate peptide (INNOVAGEN) in 300 µL of ultrapure water, then aliquoted and stored at -40°C in refrigerator. The H2-Opt working solution (50 µM) was obtained by diluting the H2-Opt stock solution with Reaction buffer B.

The recombinant human HTRA1 was diluted with Reaction buffer B to 2.016 µg/mL (40 nM) to obtain the HTRA1 enzyme working solution. The tested samples were serially diluted with Reaction buffer B.

Sixty microliters of HTRA1 enzyme working solution and 20 µL of tested samples (Control (IgG1), Galegenimab, and SA239) were sequentially added to a black low-adsorption U-bottom 96-well plate.

The positive control (used for assay evaluation, data not shown) consisted of 20 µL of Reaction buffer B and 60 µL of HTRA1 enzyme working solution.

The negative control (used for assay evaluation, data not shown) consisted of 80 µL of Reaction buffer B.

The plate was incubated on ice for 30 min. Then, 40 µL of H2-Opt working solution was added to each well. Fluorescence was measured at 30°C with excitation/emission wavelengths of 320 nm/405 nm, and data were collected every minute for 45 min. The raw data and the slopes obtained from linear fitting were exported.

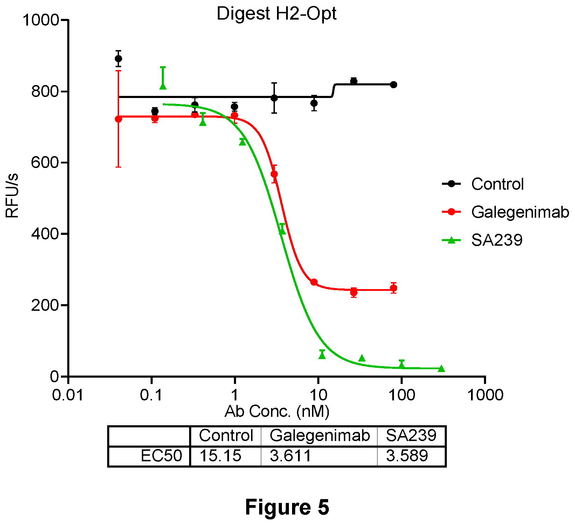

One anti-HTRA1 VHH antibody blocking result obtained in the present invention is shown in Figure 5. The candidate molecule SA239 antibody was able to block the activity of HTRA1 in enzymatic cleaving H2-Opt.

### Example 10. Elastase assay by anti-HTRA1 VHH antibodies blocking

HTRA1 is a serine protease that can cleave certain extracellular matrix components. When the substrate is elastin, HTRA1 exhibits elastase activity. The commercial SensoLyte^{®} Green Elastase Assay Kit (AnaSpec, AS-72178) was used to detect elastase activity and to verify the blocking activity of the anti-HTRA1 VHH antibody against HTRA1-mediated enzymatic cleavage of elastin.

The reagents were moved from -30°C to room temperature. The 1X Assay buffer was prepared by mixing 10 mL of Component C with 10 mL of ultrapure water. Component A was diluted 100-fold with 1X Assay buffer (freshly prepared) to obtain the Elastase substrate solution. The recombinant human HTRA1 (i.e., the recombinant human HTRA1 antigen of Example 1) was diluted with 1X Assay buffer to 20 µg/mL to obtain the HTRA1 working solution. The tested samples were serially diluted with 1X Assay buffer.

Ten microliters of tested samples (Control (IgG1), Galegenimab, and SA239) and 40 µL of HTRA1 working solution were sequentially added to a black low-adsorption U-bottom 96-well plate.

The positive control (used for assay evaluation, data not shown) consisted of 10 µL of 1X Assay buffer and 40 µL of HTRA1 working solution.

The negative control (used for assay evaluation and calculation of the sample reaction rate, data not shown) consisted of 50 µL of 1X Assay buffer.

The plate was incubated on ice for 30 min. Then, 50 µL of Elastase substrate solution was added to each well. Fluorescence was measured with excitation/emission wavelengths of 490 nm/520 nm, and data were collected every 2 min for 30 min. The slopes obtained from linear fitting were exported.

The sample reaction rate was calculated as follows:$Sample reaction rate \left(RFU/s\right) = mean of tested sample - mean of negative control$

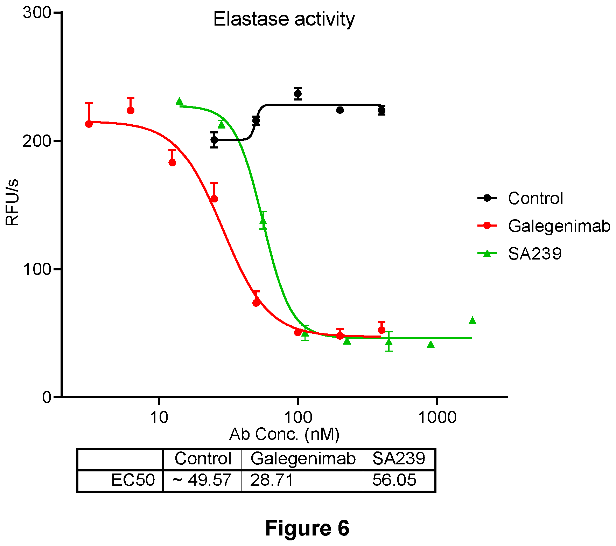

One anti-HTRA1 VHH antibody blocking result obtained in the present invention is shown in Figure 6. The candidate molecule SA239 antibody was able to block the activity of HTRA1 in cleaving elastin.

### Example 11. Humanization, activity detection, and protein expression purification of anti-C5 VHH antibodies

The present invention utilizes molecular biology techniques to obtain anti-C5 positive VHH antibody sequences, and uses the transient expression and purification thereof to obtain VHH antibody proteins.

The amino acid sequences of the heavy chain variable region of the two anti-C5 VHH antibodies (1 and 293 and LA44) obtained in the present invention, and the sequence numbers, are shown in the attached tables.

### Humanization of immune library antibodies 1 and 293

The antibody 1,293 obtained in Example 5 and Example 6 was humanized through the following steps :
**(1).** Determining CDR loop structure;
**(2).** Finding the closest homologous sequence for each V/J region of VHH in a human germline sequence database;
**(3).** Constructing the CDR region of VHH on the human framework region;
**(4).** Using sequence and structural features to identify amino acid positions in the framework region that serve to maintain CDR functions;
**(5).** Carrying out reverse mutations at sequence positions determined to be important;
**(6).** Optimizing amino acids at risk positions.

The amino acid sequences of the three humanized antibodies obtained in the present invention are shown in the attached sequence list.

The specific sequence numbers are shown in Table 3:

**Table 3: Anti-C5 VHH antibodies and CDR sequences thereof**

| Antibody No. | VHH | HCDR1 | HCDR2 | HCDR3 |
|---|---|---|---|---|
| 1 | 17 | 1 | 2 | 3 |
| 293 | 18 | 4 | 5 | 30 |
| HZ293H5.4 | 19 | 4 | 5 | 6 |
| HZ293H5.4DV | 20 | 4 | 7 | 6 |
| HZ293H5.4SS | 21 | 4 | 8 | 6 |
| hzAP02-1.H28 | 22 | 1 | 9 | 10 |
| hzAP02-1.H29 | 23 | 1 | 9 | 10 |

### Protein expression

Expi-293 cells (Thermo Fisher Scientific) were passaged according to the required transfection volume, and the cell density was adjusted to 1.5×10⁶ cells/mL one day before transfection. On the day of transfection, the cell density was approximately 3×10⁶ cells/mL. Opti-MEM medium (Gibco, 11058021) equal to 1/10 of the final volume was used as the transfection buffer, and the appropriate plasmid was added and mixed thoroughly. The appropriate amount of polyethyleneimine (PEI) (Polysciences, 23966) was added to the plasmid at a plasmid-to-PEI mass ratio of 1:3, mixed thoroughly, and incubated at room temperature for 10 min. The mixture was then added to Expi293 cells, and the cells were cultured at 36.5°C with 8% CO₂. After 24 h, FEED (100 g/L Phytone Peptone + 100 g/L Difco Select Phytone) equal to 2% of the transfection volume, glucose solution at a final concentration of 4 g/L, and VPA (Gibco, Cat. No.: 11140-050) at a final concentration of 2 mM/L were supplemented.

The culture was maintained at 36.5°C, 120 rpm, and 8% CO₂ for 6 days. The culture was then harvested, centrifuged at 4,000 rpm for 30 min, and the cell supernatant was filtered through a 0.45 µm membrane and purified by affinity chromatography.

The specific affinity chromatography purification procedure was as follows: an Amsphere^{™} A3 (JSR Life Sciences, Cat. No.: 10000327-C05) affinity chromatography column was selected. Prior to purification, the column and tubing were treated with 0.1 M NaOH for endotoxin removal for 1 h, and then the tubing and column were rinsed with distilled water. The resin was equilibrated with 5 column volumes of 1×PBS (Gibco, Cat. No.: 10010023). The collected supernatant was loaded onto the column, and the resin was washed with 10 column volumes of 1×PBS to remove non-specifically bound proteins. The resin was eluted with 5 column volumes of elution buffer (100 mM glycine, pH 2.7), and the eluate was collected. The protein pH was then adjusted to 6.0 with 2 M Tris.

The IgG1 control (Control) (heavy chain: SEQ ID NO: 31; light chain: SEQ ID NO: 37), Galegenimab (heavy chain: SEQ ID NO: 28; light chain: SEQ ID NO: 29), and Eculizumab (heavy chain: SEQ ID NO: 32; light chain: SEQ ID NO: 33) were also subjected to similar synthesis, expression, and purification.

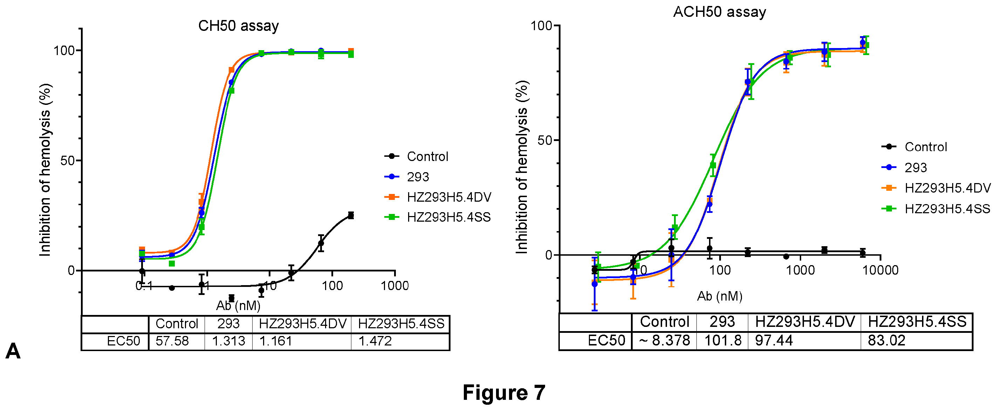

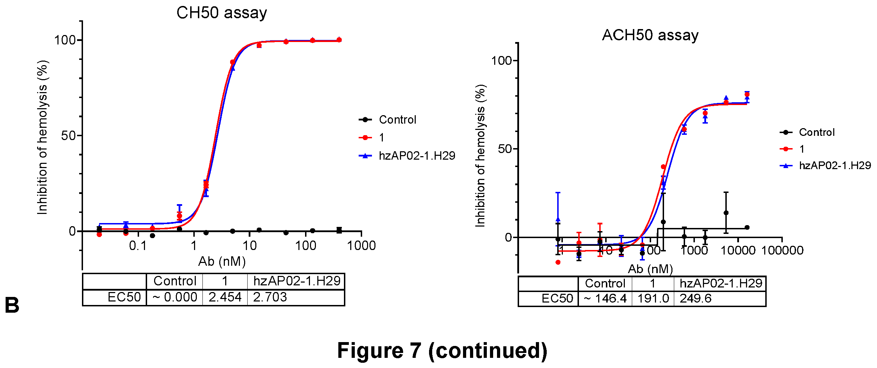

The humanized antibodies were tested using the methods described in Example 5 and Example 6 (Figure 7, Control: IgG1). The results showed that the C5 humanized antibody obtained in the present invention was able to completely inhibit C5 biological activity.

### Example 12 Affinity Maturation of Anti-HTRA1 VHH

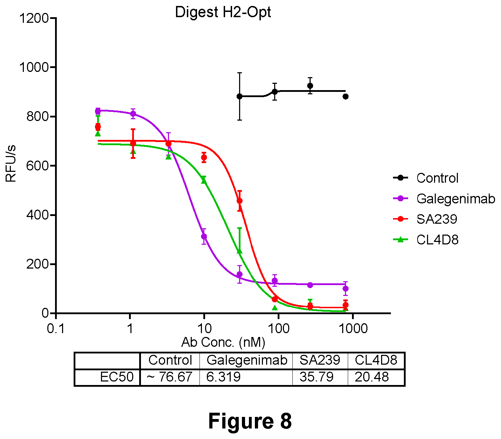

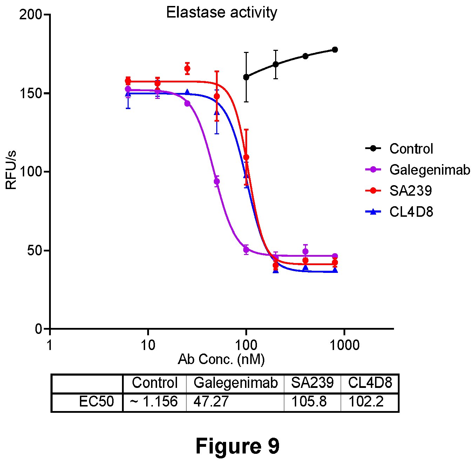

Affinity maturation was performed using a strategy in which random amino acid mutations were introduced into the antigen-binding regions (CDRs) using the SA239 VHH gene as a template. Degenerate primers containing NNK codons (Genewiz, Suzhou) and framework region-specific primers (Genewiz, Suzhou) were designed and synthesized. The antibody mutant gene library was amplified by overlap extension PCR (OE-PCR). The PCR fragments and vector were digested, ligated, and transformed into TG1 competent cells using the same method as described in Example 1 to construct the recombinant phage library. Three rounds of phage panning were performed, followed by monoclonal ELISA screening. Candidate monoclonal antibodies were expressed as VHH recombinant antibody in eukaryotic cells using the method described in Example 3, and the purified antibodies were further screened by the H2-Opt assay and Elastase assay (experimental methods and procedures were the same as those in Examples 9 and 10). The mutant with significantly improved function and affinity, designated clone CL4D8M, was ultimately selected (Figures 8 and 9, Control: IgG1).

The specific sequence numbers are listed in Table 4:

**Table 4: Anti-HTRA1 VHH Antibodies and CDR Sequences thereof**

| Antibody No. | VHH | HCDR1 | HCDR2 | HCDR3 |
|---|---|---|---|---|
| SA239 | 24 | 11 | 12 | 13 |
| CL4D8M | 25 | 14 | 15 | 16 |

### Example 13. Combination of Anti-C5 VHH Antibodies targeting Different Epitopes in Complement-Induced Hemolysis Assay

During the screening of anti-C5 VHH antibodies, clone 1 and clone 293 were able to completely block CH50 (Figure 1), but could not completely block ACH50 (Figure 2). In this experiment, anti-C5 VHH antibodies targeting different epitopes were combined to verify their blocking effect on ACH50.

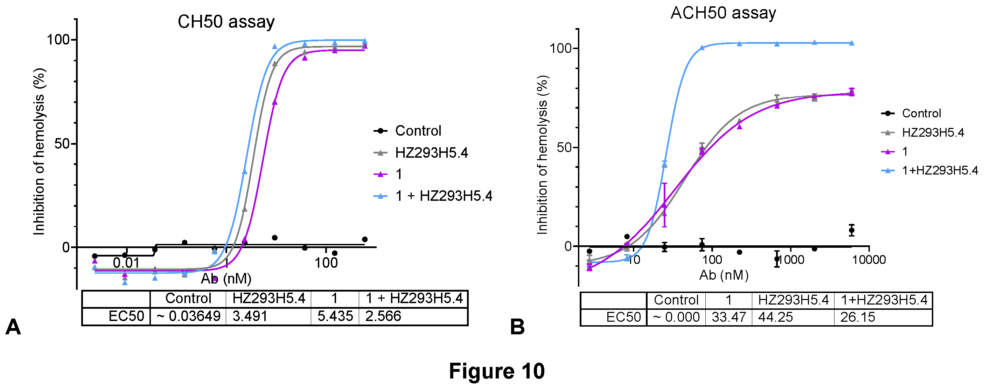

The detection methods used in this experiment were the same as those described in Example 5 and Example 6, in which HZ293H5.4 was the humanized antibody of clone 293. The results (Figures 10A and 10B) showed that the combination of anti-C5 VHH antibodies targeting different epitopes, at starting concentrations of 600 nM for clone 1 and 600 nM for HZ293H5.4 with 4-fold serial dilutions (for the CH50 assay), and at starting concentrations of 6000 nM for clone 1 and 6000 nM for HZ293H5.4 with 3-fold serial dilutions (for the ACH50 assay), could completely block the complement classical pathway (CH50) (Figure 10A) and the complement alternative pathway (ACH50) (Figure 10B), respectively.

### Example 14: Assay in which Dual-epitope Anti-C5 Antibody block Complement-Induced Hemolysis

The screened anti-C5 VHH antibodies were constructed into dual-epitope anti-C5 antibodies using GGGGGGGGGG (SEQ ID NO: 26) as a linker. In terms of molecular format, the present invention employed two anti-C5 VHH antibodies targeting different epitopes (hzAP02-1.H29 and HZ293H5.4DV) to construct bivalent and trivalent antibodies.

The configuration and sequence of the constructed B1 antibody (bivalent) were as follows:
HZ293H5.4DV-hzAP02-1.H29 (SEQ ID NO: 34), in which the two VHH domains were connected by a linker (SEQ ID NO: 26).

The configuration and sequence of the constructed B3 antibody (trivalent) were as follows:
HZ293H5.4DV - HZ293H5.4DV - hzAP02-1.H29 (SEQ ID NO: 35), in which the individual VHH domains were connected by linkers (SEQ ID NO: 26).

The configuration and sequence of the constructed B4 antibody (trivalent) were as follows:
HZ293H5.4DV - hzAP02-1.H29 - HZ293H5.4DV (SEQ ID NO: 36), in which the individual VHH domains were connected by linkers (SEQ ID NO: 26).

Their blocking activity was verified by complement-induced hemolysis assays.

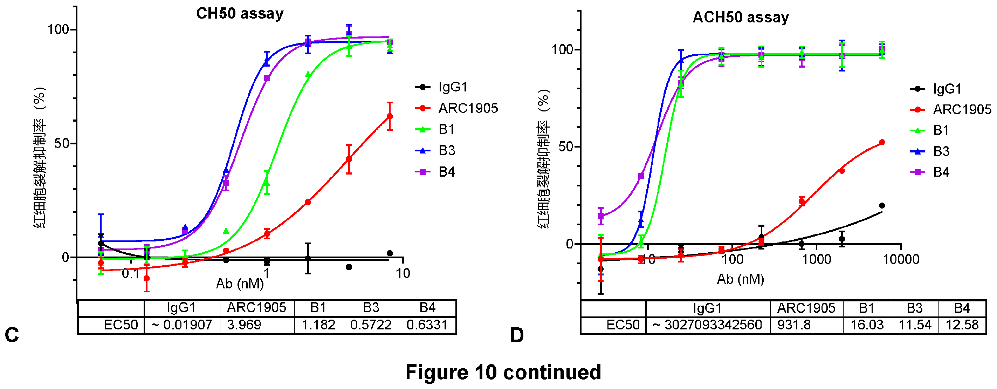

The experimental detection methods were the same as those described in Examples 5 and 6 herein (in which ARC1905 was from Biosyntech), and the results (Figures 10C and 10D) showed that the dual-epitope anti-C5 VHH antibody could completely block the complement classical pathway (Figure 10C) and the complement alternative pathway (Figure 10D).

### Example 15: Molecular Format Optimization of Anti-C5/HTRA1 Bispecific Antibody

The screened anti-C5 VHH antibody and anti-HTRA1 antibody were constructed into an anti-C5/HTRA1 bispecific antibody using GGGGGGGGGG as a linker. In terms of molecular format, the present invention employed two anti-C5 VHH domains and one anti-HTRA1 VHH domain. For the two anti-C5 VHH domains, the present invention constructed both a single epitope bivalent format of the anti-C5 VHH (two identical anti-C5 VHH domains) and a dual-epitope format of the anti-C5 VHH (two different anti-C5 VHH domains).

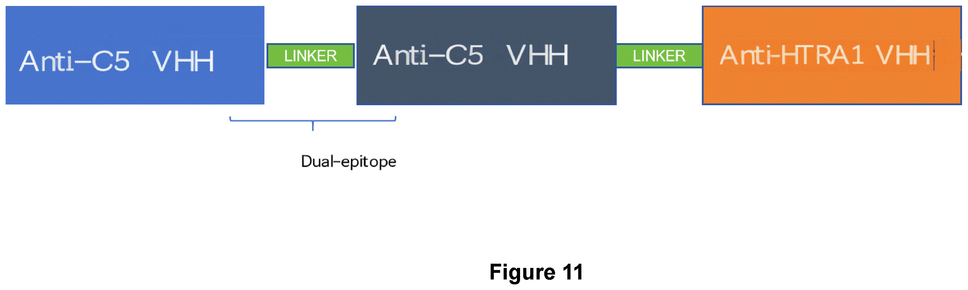

Two anti-C5 VHH antibodies (targeting different epitopes) and one anti-HTRA1 VHH antibody were constructed into a bispecific antibody, and the molecular configuration is shown in Figure 11.

The configuration and sequence of the constructed IAR045-001 antibody were as follows:
C5 HZ293H5.4D - C5 hzAP02-1.H - HTRA1 CL4D8M (SEQ ID NO: 27), in which the individual VHH domains were connected by linkers (SEQ ID NO: 26).

The binding kinetics of the IAR045-001 molecule to antigens were determined by bio-layer interferometry.

The detection methods used in this experiment were the same as those described in Example 4. Human Complement C5 (Cat. No. CO5-H52Ha), Monkey Complement C5 (Cat. No. CO5-C52Hx), Human Complement C5 (R885H) (Cat. No. CO5-H52Hx), Mouse Complement C5 (Cat. No. CO5-M52H4), Rabbit Complement C5 (Cat. No. CO5-R52H4), and Rat Complement C5 (Cat. No. CO5-R52H5) proteins were purchased from ACRO Biosystems. Dog Complement C5 (sequence reference: UniProtKB: A0A8I3NVC1, Q19-S1680), Monkey HTRA1 (sequence reference: NCBI: XP_045217639.1, Q24-P481), Mouse HTRA1 (sequence reference: UniProtKB: Q9R118, P24-P480), Rat HTRA1 (sequence reference: UniProtKB: Q9QZK5, L23-P480), Rabbit HTRA1 (sequence reference: NCBI: XP_051680286.1, full length), and Dog HTRA1 (sequence reference: UniProtKB: A0A8C0RQ32, Q30-P485) proteins were expressed in-house. Human HTRA1 (sequence reference: UniProtKB: Q92743, Q23-P480) protein was custom-ordered from Sino. The antibody affinities are shown in Table 5:

**Table 5. Affinity constants (equilibrium dissociation constants) of IAR045-001 antibody-antigen binding determined by ForteBio**

| **Antigen** | equilibrium dissociation constants | | |
|---|---|---|---|
| | **KD (M)** | **Kon(1/Ms)** | **kdis(1/s)** |
| Human Complement C5 | <2.84E-10 | 7.05E+05 | <2.00E-04 |
| Human Complement C5(R885H) | 5.59E-10 | 8.46E+05 | 4.73E-04 |
| Monkey Complement C5 | 1.05E-10 | 2.09E+06 | 2.18E-04 |
| Mouse Complement C5 | 4.46E-09 | 1.47E+06 | 6.55E-03 |
| Rat Complement C5 | <3.44E-10 | 5.81E+05 | <2.00E-04 |
| Rabbit Complement C5 | 2.08E-10 | 1.01E+06 | 2.09E-04 |
| Dog Complement C5 | No binding | | |
| Human HTRA1 | 1.58E-09 | 3.55E+05 | 5.62E-04 |
| Monkey HTRA1 | 1.31E-08 | 3.46E+05 | 4.51E-03 |
| Mouse HTRA1 | 7.75E-08 | 7.91E+05 | 6.13E-02 |
| Rat HTRA1 | No binding | | |
| Rabbit HTRA1 | 2.61E-09 | 9.73E+05 | 2.54E-03 |
| Dog HTRA1 | 2.03E-09 | 4.21E+05 | 8.53E-04 |

In the above assays, the K_{D} value of the bispecific antibody molecule IAR045-001 for human C5 was less than 2.84E-10 M, the K_{D} value for human C5 (R885H) was 5.59E-10 M, the K_{D} value for monkey C5 was 1.05E-10 M, the KD value for mouse C5 was 4.46E-09 M, the K_{D} value for rat C5 was less than 3.44E-10 M, the K_{D} value for rabbit C5 was 2.08E-10 M, and there was no binding to dog C5. The K_{D} value for human HTRA1 was 1.58E-09 M, the K_{D} value for monkey HTRA1 was 1.31E-08 M, the K_{D} value for mouse HTRA1 was 7.75E-08 M, there was no binding to rat HTRA1, the K_{D} value for rabbit HTRA1 was 2.61E-09 M, and the K_{D} value for dog HTRA1 was 2.03E-09 M.

Its blocking activity was verified by complement-induced hemolysis assays.

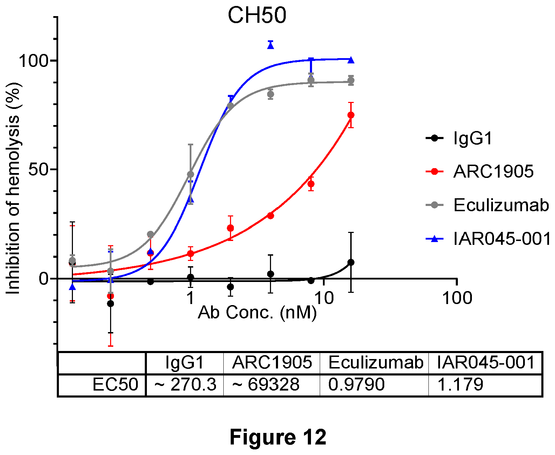

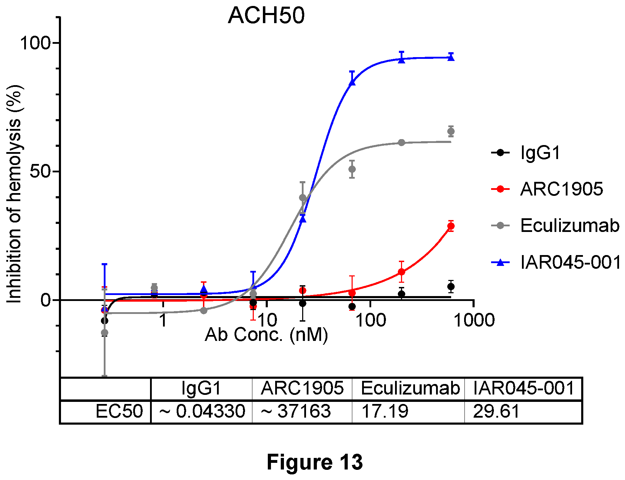

The experimental detection methods were the same as those described in Examples 5 and 6 (in which ARC1905 was from Biosyntech, Eculizumab was synthesized as described above, and Control: IgG1). The results showed that IAR045-001 could completely block the complement classical pathway (Figure 12) and the complement alternative pathway (Figure 13).

### Example 15: Blocking of HTRA1 Enzymatic Activity by Bispecific Antibody Molecule

The bispecific antibody molecule IAR045-001 contains one anti-HTRA1 VHH antibody, and its blocking activity against HTRA1 was verified by the H2-Opt assay and the Elastase assay.

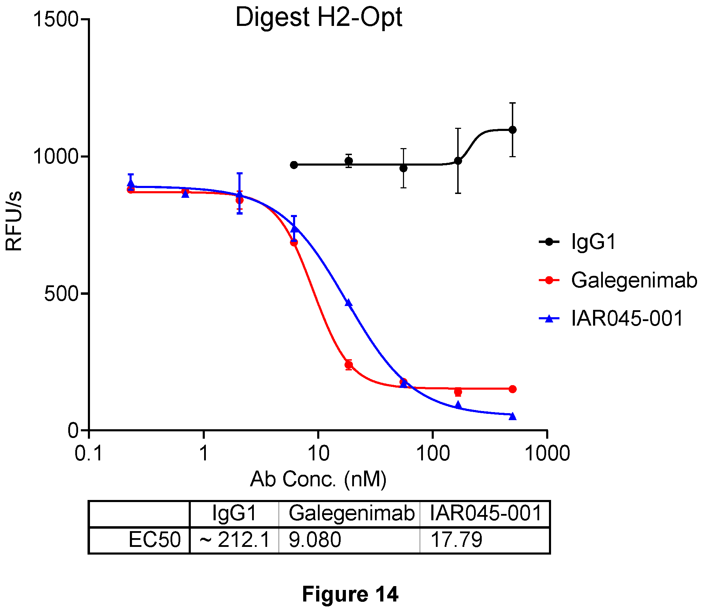

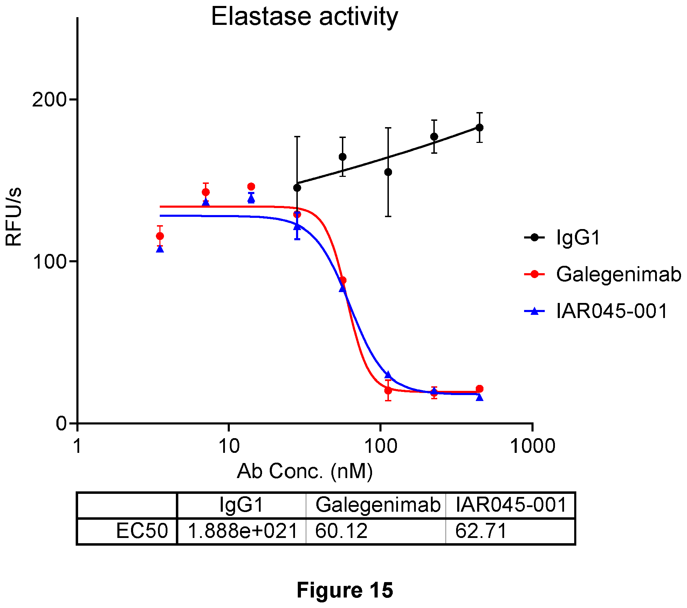

The experimental detection methods were the same as those described in Examples 9 and 10. The results showed that IAR045-001 could block the activity of HTRA1 in cleaving H2-Opt (Figure 14) and the activity of HTRA1 in cleaving elastin (Figure 15).

### Example 16: In Vivo Efficacy Study of Sodium Iodate-Induced Geographic Atrophy

The therapeutic effect of the anti-C5/HTRA1 bispecific antibody of the present invention was evaluated in a sodium iodate-induced geographic atrophy model.

The experimental animals were hC5 mice; sex: male; age: 6-8 weeks; source: Shanghai Model Organisms Center, Inc..

In this experiment, sodium iodate was administered by tail vein injection to induce damage to retinal pigment epithelial cells in mice, thereby causing phenotypes such as thinning of the outer nuclear layer (ONL) and photoreceptor outer segment (POS) regions of the retina, and a mouse model with phenotypes similar to human geographic atrophy was established. A total of 28 humanized complement C5 mice (hC5 mice) were selected and divided into 5 groups: model control group (4 mice), IgG1 group (6 mice), Galegenimab group (6 mice), ARC1905 (Biointron) group (6 mice), and IAR045-001 group (6 mice). On Day 0, sodium iodate was administered, while the model control group was not treated. On Day 0 and Day 7, tested samples were administered by intravitreal injection. On Day 10, tissues were collected, and eye tissue sections were prepared and subjected to H&E staining to analyze the inhibitory effect of the tested samples on geographic atrophy. The experimental design is shown in Table 6.

**Table 6. Design of experiments**

| Group | Dose | Administratio n Volume | Conc. | Mode of Administratio n |
|---|---|---|---|---|
| Model control group (Untreated) | / | / | / | / |
| IgG1 group | 10 µg/eye | 1 µL/eye | 10mg/m l | Intravitreal administratio n |
| Galegenimab group | 10 µg/eye | 1 µL/eye | 10mg/m l | Intravitreal administratio n |
| ARC1905 group | 10 µg/eye | 1 µL/eye | 10mg/m l | Intravitreal administratio n |
| IAR045-001 group | 10 µg/eye | 1 µL/eye | 10mg/m l | Intravitreal administratio n |

### H&E Staining Histopathological Examination

The evaluation parameters were as follows:
(1) Thickness of the retinal outer nuclear layer;
(2) Thickness of the retinal POS layer;
(3) Integrity of the retinal pigment epithelial cell layer,

The integrity of the RPE was statistically analyzed at a distance of 250 µm from the optic nerve over a length of 40 µm: brown area / total area × 100%.

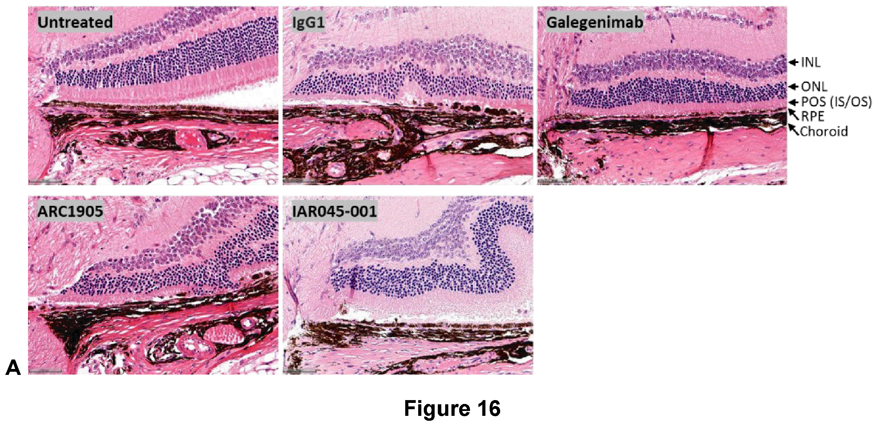

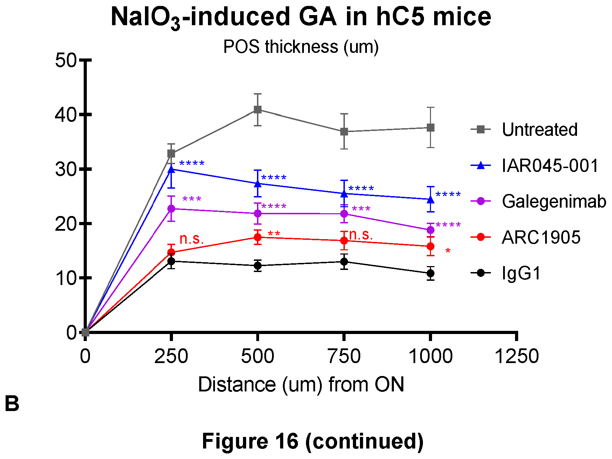

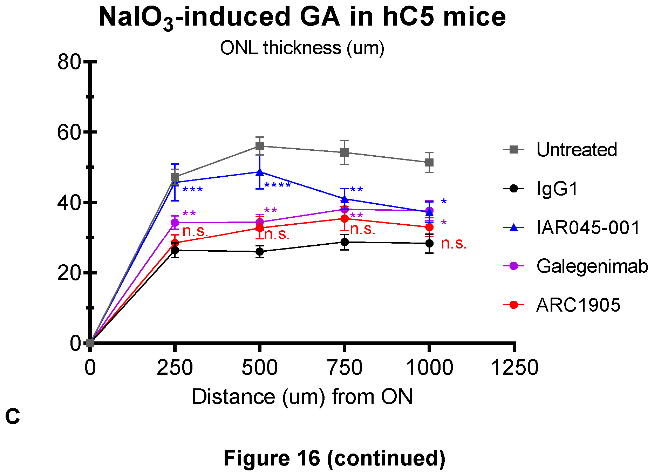

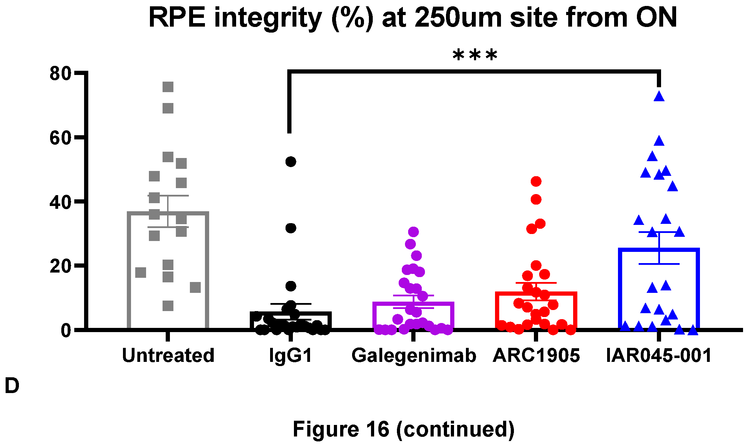

As shown in the results of Figure 16, Figure 16A presents representative H&E-stained ocular tissue images from each experimental group. The arrows in the figure sequentially mark the inner nuclear layer (INL), outer nuclear layer (ONL), photoreceptor outer segment (POS), retinal pigment epithelium (RPE), and choroid in the mouse retina. The upper left panel shows the retinal tissue of a normal mouse, with intact ONL, POS, and RPE. The upper middle panel shows the retinal tissue of the IgG1 group mouse, in which the ONL and POS layers were thinner compared to the normal mouse, and the RPE appeared discontinuous. The upper right panel shows the retinal tissue of the Galegenimab group mouse, in which the ONL and POS layers were thicker compared to the IgG1 group, and the RPE was more intact. The lower left panel shows the retinal tissue of the ARC1905 group mouse, in which the ONL and POS layers were thicker compared to the IgG1 group, and the RPE was more intact. The lower middle panel shows the retinal tissue of the IAR045-001 group mouse, in which the ONL and POS layers were thicker compared to the IgG1 group, and the RPE was more intact.

Figure 16B is a statistical plot of the POS layer thickness in the retina of mice in each group, showing that the POS thickness in the IAR045-001 group was greater than that in the IgG1, ARC1905, and Galegenimab groups.

Figure 16C is a statistical plot of the ONL layer thickness in the retina of mice in each group, showing that the ONL thickness in the IAR045-001 group was also greater than that in the IgG1, ARC1905, and Galegenimab groups.

Figure 16D is a statistical plot of the integrity of the retinal pigment epithelial cell layer in mice in each group, showing that the pigment epithelial cell layer in the IAR045-001 group was more intact than that in the IgG1, ARC1905, and Galegenimab groups.

In summary, the antibody of the present invention could significantly restore the sodium iodate-induced thinning of the ONL layer, thinning of the POS layer, and the integrity of the pigment epithelial cells, and the effect was better than that of ARC1905 and Galegenimab.

### Sequence list

| Clone No./Antibo dy Name | SEQ ID NO | Target | Sequences (HCDR1 is defined according to AbM, HCDR2 is defined according to Kabat and HCDR3 is defined according to AbM) |
|---|---|---|---|
| 1 | 17 | C5 | EVQLVESGGGLVEPGRSLRLSCAVSLDYNAIGWFRQAPG KEREGVSCKSAGGSTYYADSVKGRFTISGSNAKNTLYLR MNDLKPEDAAVYYCAARAVGNCGSTWYDPDQYAYWG QGTPVTVSS |
| HCDR1 | 1 | | LDYNAIG |
| HCDR2 | 2 | | CKSAGGSTYYADSVKG |
| HCDR3 | 3 | | RAVGNCGSTWYDPDQYAY |
| 293 | 18 | C5 | QVQLVESGGGLVQPGGSLRLSCAAAESIFRDNAMGWYR QPPGKQRELVAAVTEEGETYYADSVKGRFTTSRDNAENT VYLQMNNLNPEDTAVYYCNAIWAGDPVEDWGQGTQVI VSS |
| HCDR1 | 4 | | ESIFRDNAMG |
| HCDR2 | 5 | | AVTEEGETYYADSVKG |
| HCDR3 | 30 | | IWAGDPVED |
| HZ293H5.4 | 19 | C5 | EVQLLESGGGLVQPGGSLRLSCAASESIFRDNAMGWYR QAPGKQRELVSAVTEEGETYYADSVKGRFTISRDNSKNT LYLQMNSLRAEDTAVYYCNAIWAGEPVEDWGQGTLVT VSS |
| HCDR1 | 4 | | SEQ ID NO:4 |
| HCDR2 | 5 | | SEQ ID NO:5 |
| HCDR3 | 6 | | IWAGEPVED |
| HZ293H5.4 DV | 20 | C5 | EVQLLESGGGLVQPGGSLRLSCAASESIFRDNAMGWYR QAPGKQRELVSAVTEEGETYYADVVKGRFTISRDNSKNT LYLQMNSLRAEDTAVYYCNAIWAGEPVEDWGQGTLVT VSS |
| HCDR1 | 4 | | SEQ ID NO:4 |
| HCDR2 | 7 | | AVTEEGETYYADVVKG |
| HCDR3 | 6 | | SEQ ID NO:6 |
| HZ293H5.4 SS | 21 | C5 | EVQLLESGGGLVQPGGSLRLSCAASESIFRDNAMGWYR QAPGKQRELVSAVTEEGETYYASSVKGRFTISRDNSKNT LYLQMNSLRAEDTAVYYCNAIWAGEPVEDWGQGTLVT VSS |
| HCDR1 | 4 | | SEQ ID NO:4 |
| HCDR2 | 8 | | AVTEEGETYYASSVKG |
| HCDR3 | 6 | | SEQ ID NO:6 |
| hzAP02-1. H28 | 22 | C5 | EVQLVESGGGLIQPGRSLRLSCAASLDYNAIGWFRQAPG KGLEGVSCKSAGGSTYYAESVKGRFTISGSNAKNTLYLR MNSLRAEDTAVYYCAARAVGNCGSTWYEPDQYAYWGQ GTLVTVSS |
| HCDR1 | 1 | | SEQ ID NO:1 |
| HCDR2 | 9 | | CKSAGGSTYYAESVKG |
| HCDR3 | 10 | | RAVGNCGSTWYEPDQYAY |
| hzAP02-1. H29 | 23 | C5 | EVQLVESGGGLIQPGRSLRLSCAASLDYNAIGWFRQAPG KEREGVSCKSAGGSTYYAESVKGRFTISGSNAKNTLYLR MNSLRAEDTAVYYCAARAVGNCGSTWYEPDQYAYWGQ GTPVTVSS |
| HCDR1 | 1 | | SEQ ID NO:1 |
| HCDR2 | 9 | | SEQ ID NO:9 |
| HCDR3 | 10 | | SEQ ID NO:10 |
| SA239 | 24 | HTRA 1 | QVQLVESGGGLVQPGGSLRLSCAASGFTFRSGDMSWFR QAPGKGREFVAAITSDGSETEYADSVKGRFTISRDNAKN TLYLQMNSLRAEDTAVYYCNTVYRFPTSDDDFYEYWGQ GTLVTVSS |
| HCDR1 | 11 | | GFTFRSGDMS |
| HCDR2 | 12 | | AITSDGSETEYADSVKG |
| HCDR3 | 13 | | VYRFPTSDDDFYEY |
| CL4D8M | 25 | HTRA 1 | QVQLVESGGGLVQPGGSLRLSCAASGFTFRTGDMSWFR QAPGKGREFVAAIISAGSETEYADSVKGRFTISRDNAKNT LYLQMNSLRAEDTAVYYCNTVVEPSTTDDHFYEYWGQ GTLVTVSS |
| HCDR1 | 14 | | GFTFRTGDMS |
| HCDR2 | 15 | | AIISAGSETEYADSVKG |
| HCDR3 | 16 | | VVEPSTTDDHFYEY |
| Linker | 26 | | GGGGGGGGGG |
| IAR045-00 1 | 27 | C5 and HTRA 1 | EVQLLESGGGLVQPGGSLRLSCAASESIFRDNAMGWYR QAPGKQRELVSAVTEEGETYYADVVKGRFTISRDNSKNT LYLQMNSLRAEDTAVYYCNAIWAGEPVEDWGQGTLVT VSSGGGGGGGGGEVQLVESGGGLIQPGRSLRLSCAAS LDYNAIGWFRQAPGKEREGVSCKSAGGSTYYAESVKGR FTISGSNAKNTLYLRMNSLRAEDTAVYYCAARAVGNCG STWYEPDQYAYWGQGTPVTVSSGGGGGGGGGQVQLV ESGGGLVQPGGSLRLSCAASGFTFRTGDMSWFRQAPGK GREFVAAIISAGSETEYADSVKGRFTISRDNAKNTLYLQM NSLRAEDTAVYYCNTVVEPSTTDDHFYEYWGQGTLVTV SS EVQLVQSGAEVKKPGASVKVSCKASGYKFTDSEMHWV RQAPGQGLEWIGGVDPETEGAAYNQKFKGRATITRDTS TSTAYLELSSLRSEDTAVYYCTRGYDYDYALDYWGQGT LVTVSSASTKGPSVFPLAPSSKSTSGGTAALGCLVKDYFP EPVTVSWNSGALTSGVHTFPAVLQSSGLYSLSSVVTVPSS |
| Galegenima b - Heavy chain | 28 | HTRA 1 | EVQLVQSGAEVKKPGASVKVSCKASGYKFTDSEMHWV RQAPGQGLEWIGGVDPETEGAAYNQKFKGRATITRDTS TSTAYLELSSLRSEDTAVYYCTRGYDYDYALDYWGQGT LVTVSSASTKGPSVFPLAPSSKSTSGGTAALGCLVKDYFP EPVTVSWNSGALTSGVHTFPAVLQSSGLYSLSSVVTVPSS SLGTQTYICNVNHKPSNTKVDKKVEPKSCD |
| Galegenima b-light chain | 29 | HTRA 1 | DIQMTQSPSSLSASVGDRVTITCRASSSVEFIHWYQQKPG KAPKPLISATSNLASGVPSRFSGSGSGTDFTLTISSLQPED FATYYCQQWSSAPWTFGQGTKVEIKRTVAAPSVFIFPPSD EQLKSGTASVVCLLNNFYPREAKVQWKVDNALQSGNS QESVTEQDSKDSTYSLSSTLTLSKADYEKHKVYACEVTH QGLSSPVTKSFNRGEC |
| IgG1 control - Heavy chain | 31 | | QVQLVQSGAEVKKPGASVKVSCQASGYRFSNFVIHWVR QAPGQRFEWMGWINPYNGNKEFSAKFQDRVTFTADTSA NTAYMELRSLRSADTAVYYCARVGPYSWDDSPQDNYY MDVWGKGTTVIVSSASTKGPSVFPLAPSSKSTSGGTAAL GCLVKDYFPEPVTVSWNSGALTSGVHTFPAVLQSSGLYS LSSVVTVPSSSLGTQTYICNVNHKPSNTKVDKKVEPKSC DKTHTCPPCPAPELLGGPSVFLFPPKPKDTLMISRTPEVT CVVVDVSHEDPEVKFNWYVDGVEVHNAKTKPREEQYN STYRVVSVLTVLHQDWLNGKEYKCKVSNKALPAPIEKTI SKAKGQPREPQVYTLPPSRDELTKNQVSLTCLVKGFYPS DIAVEWESNGQPENNYKTTPPVLDSDGSFFLYSKLTVDK SRWQQGNVFSCSVMHEALHNHYTQKSLSLSPG |
| IgG1 control - Light chain | 37 | | EIVLTQSPGTLSLSPGERATFSCRSSHSIRSRRVAWYQHKP GQAPRLVIHGVSNRASGISDRFSGSGSGTDFTLTITRVEPE DFALYYCQVYGASSYTFGQGTKLERKRTVAAPSVFIFPPS DEQLKSGTASVVCLLNNFYPREAKVQWKVDNALQSGN SQESVTEQDSKDSTYSLSSTLTLSKADYEKHKVYACEVT HQGLSSPVTKSFNRGEC |
| Eculizumab - Heavy Chain | 32 | | QVQLVQSGAEVKKPGASVKVSCKASGYIFSNYWIQWVR QAPGQGLEWMGEILPGSGSTEYTENFKDRVTMTRDTST STVYMELSSLRSEDTAVYYCARYFFGSSPNWYFDVWGQ GTLVTVSSASTKGPSVFPLAPCSRSTSESTAALGCLVKDY FPEPVTVSWNSGALTSGVHTFPAVLQSSGLYSLSSVVTVP SSNFGTQTYTCNVDHKPSNTKVDKTVERKCCVECPPCP APPVAGPSVFLFPPKPKDTLMISRTPEVTCVVVDVSQEDP EVQFNWYVDGVEVHNAKTKPREEQFNSTYRVVSVLTV LHQDWLNGKEYKCKVSNKGLPSSIEKTISKAKGQPREP QVYTLPPSQEEMTKNQVSLTCLVKGFYPSDIAVEWESNG QPENNYKTTPPVLDSDGSFFLYSRLTVDKSRWQEGNVFS CSVMHEALHNHYTQKSLSLSLGK |
| Eculizumab - Light Chain | 33 | | |
| Anti-C5 antibody B1 binding dual epitopes (293-1) | 34 | | EVQLLESGGGLVQPGGSLRLSCAASESIFRDNAMGWYR QAPGKQRELVSAVTEEGETYYADVVKGRFTISRDNSKNT LYLQMNSLRAEDTAVYYCNAIWAGEPVEDWGQGTLVT VSSGGGGGGGGGGEVQLVESGGGLIQPGRSLRLSCAAS LDYNAIGWFRQAPGKEREGVSCKSAGGSTYYAESVKGR FTISGSNAKNTLYLRMNSLRAEDTAVYYCAARAVGNCG STWYEPDQYAYWGQGTPVTVSS |
| B3 (293-293-1) | 35 | C5 | EVQLLESGGGLVQPGGSLRLSCAASESIFRDNAMGWYR QAPGKQRELVSAVTEEGETYYADVVKGRFTISRDNSKNT LYLQMNSLRAEDTAVYYCNAIWAGEPVEDWGQGTLVT VSSGGGGGGGGGEVQLLESGGGLVQPGGSLRLSCA ASESIFRDNAMGWYRQAPGKQRELVSAVTEEGETYYAD VVKGRFTISRDNSKNTLYLQMNSLRAEDTAVYYCNAIW AGEPVEDWGQGTLVTVSSGGGGGGGGGEVQLVESG GGLIQPGRSLRLSCAASLDYNAIGWFRQAPGKEREGVSC KSAGGSTYYAESVKGRFTISGSNAKNTLYLRMNSLRAED TAVYYCAARAVGNCGSTWYEPDQYAYWGQGTPVTVSS |
| B4 (293-1-293) | 36 | C5 | EVQLLESGGGLVQPGGSLRLSCAASESIFRDNAMGWYR QAPGKQRELVSAVTEEGETYYADVVKGRFTISRDNSKNT LYLQMNSLRAEDTAVYYCNAIWAGEPVEDWGQGTLVT VSSGGGGGGGGGEVQLVESGGGLIQPGRSLRLSCAA SLDYNAIGWFRQAPGKEREGVSCKSAGGSTYYAESVKG RFTISGSNAKNTLYLRMNSLRAEDTAVYYCAARAVGNC GSTWYEPDQYAYWGQGTPVTVSSGGGGGGGGGEV QLLESGGGLVQPGGSLRLSCAASESIFRDNAMGWYRQA PGKQRELVSAVTEEGETYYADVVKGRFTISRDNSKNTLY LQMNSLRAEDTAVYYCNAIWAGEPVEDWGQGTLVTVSS |

## Claims

1. A VHH antibody specifically binding C5, comprising
three complementarity determining regions (CDRs) contained in the VHH of any one of SEQ ID NOs: 23, 17 or 22;
preferably, the CDR1 sequence is defined according to AbM, CDR2 is defined according to Kabat, and CDR3 is defined according to AbM.

2. The VHH antibody specifically binding C5 of claim 1, comprising complementarity determining regions (CDRs) VHH CDR1, VHH CDR2 and VHH CDR3, wherein
(i) VHH CDR1 comprises or consists of the amino acid sequence set forth in SEQ ID NO:1, VHH CDR2 comprises or consists of the amino acid sequence set forth in SEQ ID NO:9, VHH CDR3 comprises or consists of the amino acid sequence set forth in SEQ ID NO:10;
(ii) VHH CDR1 comprises or consists of the amino acid sequence set forth in SEQ ID NO:1, VHH CDR2 comprises or consists of the amino acid sequence set forth in SEQ ID NO:2, VHH CDR3 comprises or consists of the amino acid sequence set forth in SEQ ID NO:3; or
(iii) VHH CDR1 comprises or consists of the amino acid sequence set forth in SEQ ID NO:1, VHH CDR2 comprises or consists of the amino acid sequence set forth in SEQ ID NO:2 or 9, VHH CDR3 comprises or consists of the amino acid sequence set forth in SEQ ID NO:3 or 10.

3. The VHH antibody specifically binding C5 of claim 1, comprising or consisting of a heavy chain variable region, which
(i) comprises or consists of an amino acid sequence having at least 90%, 91%, 92%, 93%, 94%, 95%, 96%, 97%, 98% or 99% identity to an amino acid sequence selected from any one set forth in SEQ ID NOs: 23, 17 or 22; or
(ii) comprises or consists of an amino acid sequence selected from any one set forth in SEQ ID NOs: 23, 17 or 22.

4. A VHH antibody specifically binding C5, comprising
three complementarity determining regions (CDRs) contained in the VHH of any one of SEQ ID NOs: 20, 18, 19 or 21,
preferably, the CDR1 sequence is defined according to AbM, CDR2 is defined according to Kabat, and CDR3 is defined according to AbM.

5. The VHH antibody specifically binding C5 of claim 4, comprising complementarity determining regions (CDRs) VHH CDR1, VHH CDR2 and VHH CDR3, wherein
(i) VHH CDR1 comprises or consists of the amino acid sequence set forth in SEQ ID NO:4, VHH CDR2 comprises or consists of the amino acid sequence set forth in SEQ ID NOs:7 or 8, VHH CDR3 comprises or consists of the amino acid sequence set forth in SEQ ID NO:6;
(ii) VHH CDR1 comprises or consists of the amino acid sequence set forth in SEQ ID NO:4, VHH CDR2 comprises or consists of the amino acid sequence set forth in SEQ ID NO:5, VHH CDR3 comprises or consists of the amino acid sequence set forth in SEQ ID NO:6 or 30; or
(iii) VHH CDR1 comprises or consists of the amino acid sequence set forth in SEQ ID NO:4, VHH CDR2 comprises or consists of the amino acid sequence set forth in SEQ ID NOs:5, 7 or 8, VHH CDR3 comprises or consists of the amino acid sequence set forth in SEQ ID NOs:30 or 6.

6. The VHH antibody specifically binding C5 of claim 4, comprising or consisting of a heavy chain variable region, which
(i) comprises or consists of an amino acid sequence that is at least 90%, 91%, 92%, 93%, 94%, 95%, 96%, 97%, 98% or 99% identical to an amino acid sequence selected from any one set forth in SEQ ID NOs: 20, 18, 19 or 21; or
(ii) comprises or consists of an amino acid sequence selected from any one set forth in SEQ ID NOs: 20, 18, 19 or 21.

7. A heavy chain antibody specifically binding C5, comprising the VHH antibody according to any one of claims 1-6.

8. The heavy chain antibody specifically binding C5 of claim 7, comprising or consisting of the VHH antibody specifically binding C5 of any one of claims 1-6 linked to an antibody constant region or Fc region.

9. A VHH antibody specifically binding HTRA1 comprising
three complementarity determining regions (CDRs) contained in the VHH set forth in SEQ ID NO: 25 or 24
preferably, the CDR1 sequence is defined according to AbM, CDR2 is defined according to Kabat, and CDR3 is defined according to AbM.

10. The VHH antibody specifically binding HTRA1 of claim 9, comprising complementarity determining regions (CDRs) VHH CDR1, VHH CDR2 and VHH CDR3, wherein
(i) VHH CDR1 comprises or consists of the amino acid sequence set forth in SEQ ID NO:14, VHH CDR2 comprises or consists of the amino acid sequence set forth in SEQ ID NO:15, VHH CDR3 comprises or consists of the amino acid sequence set forth in SEQ ID NO:16; or
(ii) VHH CDR1 comprises or consists of the amino acid sequence set forth in SEQ ID NO:11, VHH CDR2 comprises or consists of the amino acid sequence set forth in SEQ ID NO:12, VHH CDR3 comprises or consists of the amino acid sequence set forth in SEQ ID NO:13; or
(iii) VHH CDR1 comprises or consists of the amino acid sequence set forth in SEQ ID NO:11 or 14, VHH CDR2 comprises or consists of the amino acid sequence set forth in SEQ ID NO:12 or 15, VHH CDR3 comprises or consists of the amino acid sequence set forth in SEQ ID NO:13 or 16.

11. The VHH antibody specifically binding HTRA1 of claim 9, comprising or consisting of a heavy chain variable region, which
(i) comprises or consists of an amino acid sequence having at least 90%, 91%, 92%, 93%, 94%, 95%, 96%, 97%, 98% or 99% identity to amino acid sequence selected from any one set forth in SEQ ID NOs: 25 or 24; or
(ii) comprises or consists of amino acid sequence selected from any one set forth in SEQ ID NOs: 25 or 24.

12. A heavy chain antibody specifically binding HTRA1 comprising the VHH antibody specifically binding HTRA1 according to any one of claims 9-11.

13. The heavy chain antibody specifically binding HTRA1 of claim 12, comprising or consisting of the VHH antibody specifically binding HTRA1 of any one of claims 9-11 linked to an antibody constant region or Fc region.

14. The heavy chain antibody specifically binding C5 of claim 8 or the heavy chain antibody specifically binding HTRA1 of claim 13, wherein the antibody constant region or Fc region is from human IgG1, human IgG2, human IgG3 or human IgG4.

15. The VHH antibody specifically binding C5 of any one of claims 1-6, or the heavy chain antibody specifically binding C5 of claims 7 or 8, or the VHH antibody specifically binding HTRA1 of any one of claims 9-11, or the heavy chain antibody specifically binding HTRA1 of claims 12 or 13, or the heavy chain antibody specifically binding C5 or the heavy chain antibody specifically binding HTRA1 of claim 14, wherein the antibody is a chimeric antibody or a humanized antibody.

16. An antibody specifically binding C5 comprising two or three or more antigen-binding regions that specifically bind C5, wherein the antigen-binding regions bind distinct epitopes and comprise the VHH antibody specifically binding C5 of any one of claims 1-6 and 15, or the heavy chain antibody specifically binding C5 of any one of claims 7-8 and 14-15, respectively.

17. The antibody specifically binding C5 of claim 16, comprising 1 or 2 VHH antibodies or heavy chain antibodies comprising the VHH antibody specifically binding C5 according to any one of claims 1-3, and 1 or 2 VHH antibodies or heavy chain antibodies comprising the VHH antibody specifically binding C5 according to any one of claims 4-6, preferably, comprising 1 or 2 VHH antibodies that specifically binding C5 according to any one of claims 4-6, and 1 VHH antibody specifically binding C5 according to any one of claims 1-3, preferably, the VHH antibodies or the heavy chain antibodies are connected in tandem, preferably via a linker;
preferably, the antibody comprises or consists of a chain comprising from the N-terminus to the C-terminus:
(1) the VHH antibody specifically binding C5 of any one of claims 4-6 - the VHH antibody specifically binding C5 of any one of claims 1-3,
(2) the VHH antibody specifically binding C5 of any one of claims 4-6 - the VHH antibody specifically binding C5 of any one of claims 4-6 - the VHH antibody specifically binding C5 of any one of claims 1-3, or
(3) the VHH antibody specifically binding C5 of any one of claims 4-6 - the VHH antibody specifically binding C5 of any one of claims 1-3 - the VHH antibody specifically binding C5 of any one of claims 4-6;
preferably, the VHHs are linked by a linker.

18. A multispecific antibody comprising one or more antigen-binding regions which specifically bind C5 and one or more other antigen-binding regions, where the antigen-binding region specifically binding C5 comprises the VHH antibody specifically binding C5 of any one of claims 1-6 and 15 or the heavy chain antibody specifically binding C5 of any one of claims 7-8 and 14-15, preferably, the multispecific antibody is a bispecific antibody.

19. The multispecific antibody of claim 18 further comprising one or more antigen-binding regions which specifically bind HTRA1, for example the antigen-binding region which specifically bind HTRA1 comprises the VHH antibody specifically binding HTRA1 of any one of claims 9-11 and 15, or the heavy chain antibody specifically binding HTRA1 of any one of claims 12-15.

20. A multispecific antibody comprising an antigen-binding region specifically binding HTRA1 and one or more other antigen-binding regions, wherein the antigen-binding region specifically binding HTRA1 comprises the VHH antibody specifically binding HTRA1 of any one of claims 9-11 and 15, or the heavy chain antibody specifically binding HTRA1 of any one of claims 12-15, preferably, the multispecific antibody is a bispecific antibody.

21. The multispecific antibody of claim 20 further comprising one or more antigen-binding regions which specifically bind C5, wherein the antigen-binding regions which specifically bind C5 comprise the VHH antibody specifically binding C5 of any one of claims 1-6 and 15, or the heavy chain antibody specifically binding C5 of any one of claims 7-8 and 14-15.

22. A bispecific antibody comprising one or more antigen-binding regions which specifically bind C5 and one or more antigen-binding regions which specifically bind HTRA1, wherein the antigen-binding regions which specifically bind C5 bind different epitopes on C5.

23. The bispecific antibody of claim 22 comprising two antigen-binding regions which specifically bind C5 and one antigen-binding region specifically binding HTRA1, wherein the two antigen-binding regions which specifically bind C5 bind different epitopes on C5.

24. The bispecific antibody of claim 23 wherein the two antigen-binding regions specifically binding C5 are VHH specifically binding different epitopes of C5 and the antigen-binding region specifically binding HTRA1 is VHH specifically binding HTRA1, wherein the 3 VHHs are connected in tandem, optionally the VHHs are linked together by a linker.

25. The bispecific antibody of claim 24 wherein the linker comprises (G) ₙ, where n = 5-10, for example 5, 6, 7, 8, 9, or 10.

26. The bispecific antibody of claim 22 or 23 wherein the VHHs are linked to each other via an N-terminus to an N-terminus, a C-terminus to a C-terminus, or an N-terminus to a C-terminus, optionally via a linker.

27. The bispecific antibody of any one of claims 24-26 wherein the one antigen-binding region specifically binding C5 comprises or consists of the VHH of any one of claims 1-3, the other antigen-binding region specifically binding C5 comprises or consists of the VHH of any one of claims 4-6 or the antigen-binding region specifically binding HTRA1 comprises or consists of the VHH of any one of claims 9-11.

28. The bispecific antibody of any one of claims 24-26 wherein the one antigen-binding region specifically binding C5 comprises or consists of the VHH of any one of claims 1-3, the other antigen-binding region specifically binding C5 comprises or consists of the VHH of any one of claims 4-6, and the antigen-binding region specifically binding HTRA1 comprises or consists of the VHH of any one of claims 9-11.

29. The bispecific antibody of any one of claims 24-28 comprising a chain which comprises, from N-terminus to C-terminus:
VHH1 specifically binding C5-VHH2 specifically binding C5-VHH3 specifically binding HTRA1,
the VHHs are linked by a linker;
wherein VHH1 comprises or consists of the VHH of any one of claims 4-6, VHH2 comprises or consists of the VHH of any one of claims 1-3, and VHH3 comprises or consists of the VHH of any one of claims 9-11.

30. The bispecific antibody of claim 29 comprising or consisting of a chain wherein the chain comprises or consists of the amino acid sequence set forth in SEQ ID NO:27 or an amino acid sequence having at least 90%, 91%, 92%, 93%, 94%, 95%, 96%, 97%, 98% or 99% identity thereto.

31. A nucleic acid molecule encoding the VHH antibody specifically binding C5 of any one of claims 1-6 and 15, or the heavy chain antibody specifically binding C5 of any one of claims 7 or 8 or 15, or the VHH antibody specifically binding HTRA1 of any one of claims 9-11, or the heavy chain antibody specifically binding HTRA1 of any one of claims 12 or 13 or 15, or the heavy chain antibody specifically binding C5 or the heavy chain antibody specifically binding HTRA1 of any one of claims 14 or 15; or the multispecific antibody of any one of claims 17-20, or the bispecific antibody of any one of claims 21-28.

32. An expression vector comprising the nucleic acid molecule according to claim 31, preferably, the expression vector is pCDNA3.1.

33. A host cell comprising the nucleic acid molecule according to claim 31 or the expression vector according to claim 32, preferably, the host cell is a prokaryotic or eukaryotic, for example, a CHO cell, a 293 cell, for example, an Expi293 cell.

34. A method of producing the antibody of any one of claims 1-30, comprising culturing the host cell of claim 33 under conditions suitable for the expression of the antibody or fusion protein, and optionally further comprising isolating the protein from the host cell or the host cell culture medium, and/or purifying the protein.

35. An immunoconjugate comprising the antibody of any one of claims 1-30, and one or more other active ingredients.

36. A pharmaceutical composition or a medicament or a formulation comprising the antibody of any one of claims 1-30 or the immunoconjugate of claim 35, and optionally a pharmaceutical excipient.

37. A pharmaceutical combination product, comprising
the antibody of any one of claims 1-30 or the immunoconjugate of claim 35; and
other therapeutic agents.

38. A method of preventing or treating an ocular disease or condition in a subject, comprising administering to the subject an effective amount of the antibody of any one of claims 1-30; or the immunoconjugate of claim 35 or the pharmaceutical composition or formulation of claim 36; or the pharmaceutical combination product of claim 37.

39. The method of claim 38, wherein the disease or condition refers to a disease or condition in which complement C5 protein and/or HTRA1 protein is present (e.g., at an elevated level, e.g., of nucleic acid or protein) in the subject (e.g., as compared to a healthy subject) or in a sample, e.g., a body fluid, of the subject (e.g., as compared to a body fluid of a healthy subject).

40. A method for detecting the presence of complement C5 and/or HTRA1 in a biological sample comprising contacting a biological sample with the antibody of any one of claims 1-30 under conditions that allow binding thereof to complement C5 and/or HTRA1 and detecting whether a complex is formed between the antibody and complement C5 and/or HTRA1, wherein the formation of the complex is indicative of the presence of complement C5.
